# EUROPEAN PATENT APPLICATION

(11) **EP 0 747 059 A1**
(43) Date of publication of application: **11.12.1996**
(21) Application number: 95906526.9
(22) Date of filing: 26.01.1995
(51) Int. Cl.: A61K 39/395, C07K 16/18, C12P 21/08

(54) **ANTI-INFLAMMATORY CONTAINING MONOCLONAL ANTIBODIES HAVING REACTIVITY WITH SIALYL-LEWIS X SUGAR CHAINS ORIGINATING IN HEMANGIOENDOTHELIAL CELL MEMBRANE**

(30) Priority: 28.01.1994 JP 26334/94; 08.09.1994 JP 242328/94
(71) Applicant: Japan Tobacco Inc., Minato-Ku Tokyo 105 (JP)
(72) Inventor: KANNAGI, Reiji, Nagoya-shi Aichi 464 (JP); SUEMATSU, Makoto, Tokyo 160 (JP); TAMATANI, Takuya, Pharmaceutical Basic Research, Kanazawa-ku, Yokohama-shi, Kanagawa 236 (JP)
(74) Representative: von Kreisler, Alek, Dipl.-Chem.
(86) International application number: JP9500094
(87) International publication number: WO9520400

(57) **Abstract**

A leukocyte rolling inhibitor, leukocyte adhesion inhibitor, leukocyte tissue infiltration inhibitor, and anti-inflammatory, each containing monoclonal antibodies having a reactivity with sialyl-Lewis X sugar chains originating in the hemangioendothelial cell membranes of nonlymphatic tissues (e.g., brain, trachea, lung, liver, heart, pancreas, intestine, mesentery, kidney, skin or joint), preferably further sialyl-Lewis X sugar chains originating in the cell membranes of leukocytes. The above monoclonal antibodies have not only all of the anti-inflammatory effects of the conventional monoclonal antibodies against each of L-, E- and P-selectin proteins, but also the effect of inhibiting the earliest stages of local tissue inflammation, i.e., the rolling and adhesion of leukocytes on a hemangioendothelial cell membrane and the infiltration of leukocytes into extravascular tissues. Therefore the invention provides a potent anti-inflammatory having a novel action mechanism.

## Description

### Technical Field

The present invention relates to a leukocyte rolling inhibitor comprising a monoclonal antibody having reactivity to sialyl Lewis X sugar chains derived from vascular endothelial cell membranes of nonlymphoid tissues, a leukocyte adhesion inhibitor comprising said monoclonal antibody, an inhibitor of infiltration of leukocytes into tissues, which comprises said monoclonal antibody, and to an anti-inflammatory agent comprising said monoclonal antibody.

### Background Art

Extravascular infiltration of leukocytes constantly occurs in high endothelial venules (hereinafter to be referred to as HEV) of lymph nodes of healthy living organisms through adhesion onto said HEV cell membrane. It has been known that adhesion and extravascular infiltration of leukocytes constantly occur in the same manner in a series of inflammatory responses, besides the above-mentioned adhesion and infiltration. Specifically, when some damage is given to local tissues of a living organism due to some external or internal factors, leukocytes which usually flow in the blood within blood vessels at high speeds quickly migrate to the vascular endothelial cell membrane (① migration of leukocytes), adhere (tethering) to the vascular endothelial cell membrane via adhesion molecules generally called selectin (② initial adhesion), and roll on the vascular endothelial cell membrane (③ rolling). The leukocytes strongly adhere to vascular endothelial cell membrane via various other adhesion molecules such as integrin (④ strong adhesion), infiltrate into extravascular tissues from the gap between vascular endothelial cells (⑤ infiltration) and migrate to traumatic tissue region (⑥ migration) to complete a series of inflammatory responses.

Of these processes, particularly in the process from ① migration of leukocytes to ③ rolling, namely, the process wherein the leukocytes to adhere are identified and selected from those in the blood stream flowing at an extremely high speed, and led to vascular endothelial cells, and rolled on the vascular endothelial cells, membrane proteins generally called selectin and their ligands play an important role [Lawrence MB et al., Cell, vol. 65, pp. 859-873 (1991)].

Selectin includes three kinds of L-selectin (i.e., LAM-1, LECAM-1 and Mel-14) which is expressed on leukocyte cell membranes, E-selectin (i.e., ELAM-1) which is expressed on vascular endothelial cell membranes and P-selectin (i.e., GMP-140, PADGEM and CD62) which is expressed on vascular endothelial cell membrane or platelet cell membrane.

With an increasingly clarified involvement of such selectins in leukocyte adhesion to and rolling on vascular endothelial cell membrane, an anti-inflammatory effect has been expected which is achieved by controlling the leukocyte adhesion to and rolling on vascular endothelial cell membrane via selectins. An anti-inflammatory effect by the use of monoclonal antibodies to L-, E- or P-selectin protein has been already confirmed in inflammatory models with peritonitis, pneumonia, diabetes, asthma, reperfusion injury after ischemia, burns or multiple organ failure, and the efficacies thereof have been documented [L-selectin: Watson, S.R. et al., Nature, vol. 349, pp. 164-167 (1991) and Yang, X-D et al., Proc. Natl. Acad. Sci., U.S.A., vol. 90, pp. 10494-10498 (1993); E-selectin: Mulligan, M.S. et al., J. Clin. Invest., vol. 88, pp. 1396-1406 (1991) and Gundeel, R.H. et al., J. Clin. Invest., vol. 88, pp. 1407-1411 (1991); P-selectin: Weyrich, A.S. et al., J. Clin. Invest., vol. 91, pp. 2620-2629 (1993) and Winn, R. et al., J. Clin. Invest., vol. 92, pp. 2042-2047 (1993)].

However, since the above-mentioned three kinds of selectins are concomitantly responsible for the leukocyte adhesion to and rolling on vascular endothelial cell membrane, a separate use of either monoclonal antibody to L-selectin protein, monoclonal antibody to E-selectin protein or monoclonal antibody to P-selectin protein as an anti-inflammatory agent would not provide sufficient anti-inflammatory effect.

In recent years, there has been documented that one of the *in vivo* ligands for E-selectin and P-selectin is a sugar chain antigen called sialyl Lewis X (SLeX) which is expressed on the cell membrane of leukocytes, and that the antibody having reactivity to such sialyl Lewis X (anti-sialyl Lewis X antibody) is exemplified by the antibody (SNH-3) disclosed in Japanese Patent Unexamined Publication No. 103190/1991, the antibody (CSLEX-1) disclosed in Japanese Patent Unexamined Publication No. 63700/1986, the antibody disclosed in WO 92/01718 and FH-6 [Handa et al., Biochem. Biophys. Res. Commun., vol. 181, pp. 1223-1230 (1991); Paavonen et al., Am. J. Pathol., vol. 141, pp. 1259-1264 (1992)].

Yet, there is no report on the effect to inhibit leukocyte rolling on or adhesion to vascular endothelial cell membrane, the effect to inhibit infiltration of leukocytes into tissues, or the confirmation of anti-inflammatory effect of these known anti-sialyl Lewis X antibodies.

On the other hand, with regard to the *in vivo* ligands for L-selectin which are located on vascular endothelial cell membrane, one of them is assumed to be sialyl Lewis X expressed on vascular endothelial cell membrane, based on the findings that L-selectin binds with sialyl Lewis X and that sialyl Lewis X is expressed on the HEV cell membrane of certain lymph nodes, whereas the presence of sialyl Lewis X has not been confirmed on the vascular endothelial cell membrane of various tissues, so that the conclusion is nothing more than an assumption [Tamatani, Mebio, vol. 10, No. 5, pp. 26-31 (1993); Suzuki, Mebio, vol. 10, No. 5, pp. 32-42 (1993)].

It follows therefrom that how the L-selectin on the leukocyte cell membrane is involved in rolling on or adhesion to vascular endothelial cell membrane is unknown, and clarification thereof is desired.

Under the circumstances, the present inventors have found that the monoclonal antibody prepared by them has reactivity to not only sialyl Lewis X on human leukocyte cell membrane, but also to vascular endothelial cells of lymphoid tissues such as human lymph nodes, tonsil, appendix and mesenteric lymphoid tissues, notwithstanding the fact that any known antibody shows reactivity only to sialyl Lewis X sugar chain on the cell membrane of leukocytes. This is the first finding of the presence of sialyl Lewis X on vascular endothelial cell membranes of lymphoid tissues, like the cell membrane of human leukocytes [Kannagi et al., Proceeding of the Japanese Society for Immunology, vol. 22, 3D4 (1992); Kannagi et al., Biochem. Biophys. Res. Commun., vol. 193, No. 1, pp. 337-347 (1993)].

The finding with regard to the monoclonal antibody is only that it has an effect of inhibiting leukocyte adhesion to HEV cells of lymph node where extravascular infiltration of leukocytes occurs constantly in healthy living organisms, irrespective of inflammatory responses at local tissues, but the action at local tissues other than lymph node (nonlymphoid tissues) has not been elucidated or cannot be even speculated (report *supra*).

### Disclosure of the Invention

An object of the present invention is to provide a leukocyte rolling inhibitor comprising a monoclonal antibody having reactivity to sialyl Lewis X sugar chains derived from vascular endothelial cell membranes of nonlymphoid tissues, a leukocyte adhesion inhibitor comprising said monoclonal antibody, an inhibitor of infiltration of leukocytes into tissues, which comprises said monoclonal antibody, and an anti-inflammatory agent comprising said monoclonal antibody.

Under the above-mentioned technical background, the present inventors have further conducted intensive studies with respect to the monoclonal antibody they prepared, and found that said monoclonal antibody has reactivity to not only sialyl Lewis X on leukocytes or on lymphoid tissues, but also to sialyl Lewis X sugar chains on vascular endothelial cell membranes of nonlymphoid tissues. Further studies have revealed that said antibody exhibits superior anti-inflammatory effects by strongly inhibiting leukocyte rolling on or adhesion to vascular endothelial cell membranes of nonlymphoid tissues, as well as leukocyte infiltration into tissues, which finding having been heretofore unknown, which resulted in the completion of the invention.

Accordingly, the present invention provides a leukocyte rolling inhibitor comprising a monoclonal antibody which is characterized by at least one of the following (1) to (4).
(1) Having reactivity to sialyl Lewis X sugar chains derived from vascular endothelial cell membranes of nonlymphoid tissues.
(2) Having reactivity to sialyl Lewis X sugar chains derived from vascular endothelial cell membranes of at least one nonlymphoid tissue selected from the group consisting of brain, trachea, lung, liver, heart, pancreas, intestine, mesentery, kidney, skin and joint, preferably from the group consisting of lung, liver, heart, pancreas, kidney, joint and skin.
(3) Having reactivity to sialyl Lewis X sugar chains derived from cell membranes of leukocytes, in addition to the characteristics of (1) and (2).
(4) Being produced by the hybridoma identified by international deposit No. FERM BP-4525.

The present invention provides a leukocyte adhesion inhibitor comprising a monoclonal antibody characterized by at least one of (1) to (4) above.

The present invention also provides an inhibitor of infiltration of leukocytes into tissues, which comprises a monoclonal antibody characterized by at least one of (1) to (4) above.

The present invention further provides an anti-inflammatory agent comprising a monoclonal antibody characterized by at least one of (1) to (4) above, an anti-inflammatory agent for acute inflammation which comprises said monoclonal antibody, an anti-inflammatory agent for inflammation in brain, trachea, blood vessel, lung, liver, heart, pancreas, intestine, mesentery, kidney, skin or joint, which comprises said monoclonal antibody, and an anti-inflammatory agent for inflammation associated with reperfusion injury after ischemia, immune rejection after transplantation, burns or multiple organ failure, which comprises said monoclonal antibody.

### Brief Explanation of the Drawings

In Fig. 1, A shows reactivity of monoclonal antibody 2H5 to sialyl Lewis X sugar chain, and B shows reactivity of monoclonal antibody 2H5 to sialyl Lewis **a** sugar chain, wherein the vertical line of each graph is reactivity (%) of monoclonal antibody 2H5 and the horizontal axis is concentration (ng/well) of sialyl Lewis X sugar chain or sialyl Lewis **a** sugar chain, ▲-▲ is reactivity of monoclonal antibody 2H5, ○-○ is reactivity of monoclonal antibody SNH-3, and △-△ is reactivity of monoclonal antibody 2D3.

Fig. 2 is a photograph of electrophoresis which shows, in the place of a drawing, the reactivity of monoclonal antibody 2H5 to sialyl Lewis X on cell membrane protein of human peripheral lymph node stroma by SDS polyacrylamide gel electrophoresis, wherein lane 1 is electrophoresis of solublilized protein, lane 2 is electrophoresis showing the reactivity of monoclonal antibody SNH-3, and lane 3 is electrophoresis showing the reactivity of monoclonal antibody 2H5.

Fig. 3 is a graph showing the reactivity of monoclonal antibody 2H5 to sialyl Lewis X on cell membrane of rat leukocytes (neutrophil), wherein the vertical line is relative cell counts and the horizontal axis is fluorescence intensity (logarithmic).

Fig. 4 is a graph showing the reactivity of isotype matched mouse IgM (control) to sialyl Lewis X on cell membrane of rat leukocytes (neutrophil) when it was used instead of monoclonal antibody 2H5, wherein the vertical line is relative cell counts and the horizontal axis is fluorescence intensity (logarithmic).

Fig. 5 is a graph showing the reactivity of monoclonal antibody 2H5 to sialyl Lewis X on cell membrane of human leukocytes (monocyte), wherein the vertical line is relative cell counts and the horizontal axis is fluorescence intensity (logarithmic).

Fig. 6 is a graph showing the reactivity of isotype matched mouse IgM (control) to sialyl Lewis X on cell membrane of human leukocytes (monocyte) when it was used instead of monoclonal antibody 2H5, wherein the vertical line is relative cell counts and the horizontal axis is fluorescence intensity (logarithmic).

Fig. 7 is a graph showing the reactivity of monoclonal antibody 2H5 to sialyl Lewis X on cell membrane of human leukocytes (granulocyte), wherein the vertical line is relative cell counts and the horizontal axis is fluorescence intensity (logarithmic).

Fig. 8 is a graph showing the reactivity of isotype matched mouse IgM (control) to sialyl Lewis X on cell membrane of human leukocytes (granulocyte) when it was used instead of monoclonal antibody 2H5, wherein the vertical line is relative cell counts and the horizontal axis is fluorescence intensity (logarithmic).

Fig. 9 is a microscopic photograph (magnification ×25) showing the reactivity of monoclonal antibody 2H5 to sialyl Lewis X on vascular endothelial cells of peripheral lymph node, wherein the part (one example) indicated with an arrow is stained deep.

Fig. 10 is a microscopic photograph (magnification ×25) of a section of peripheral lymph node when mouse IgM (control) was used as a primary antibody instead of monoclonal antibody 2H5.

Fig. 11 is a microscopic photograph (magnification ×25) showing the reactivity of monoclonal antibody 2H5 to sialyl Lewis X on vascular endothelial cells of mesenteric lymph node, wherein the part (one example) indicated with an arrow is stained deep.

Fig. 12 is a microscopic photograph (magnification ×50) showing the reactivity of monoclonal antibody 2H5 to sialyl Lewis X on vascular endothelial cells of liver, wherein the part (one example) indicated with an arrow is stained deep.

Fig. 13 is a microscopic photograph (magnification ×100) showing the reactivity of monoclonal antibody 2H5 to sialyl Lewis X on vascular endothelial cells of liver, wherein the part (one example) indicated with an arrow is stained deep.

Fig. 14 is a microscopic photograph (magnification ×50) of a section of liver when mouse IgM (control) was used as a primary antibody instead of monoclonal antibody 2H5.

Fig. 15 is a microscopic photograph (magnification ×10) showing the reactivity of monoclonal antibody 2H5 to sialyl Lewis X on vascular endothelial cells of kidney (medulla), wherein the part (one example) indicated with an arrow is stained deep.

Fig. 16 is a microscopic photograph (magnification ×100) showing the reactivity of monoclonal antibody 2H5 to sialyl Lewis X on vascular endothelial cells of kidney (medulla), wherein the part (one example) indicated with an arrow is stained deep.

Fig. 17 is a microscopic photograph (magnification ×10) of a section of kidney (medulla) when mouse IgM (control) was used as a primary antibody instead of monoclonal antibody 2H5.

Fig. 18 is a microscopic photograph (magnification ×10) showing the reactivity of monoclonal antibody 2H5 to sialyl Lewis X on vascular endothelial cells of kidney (cortex), wherein the part (one example) indicated with an arrow is stained deep.

Fig. 19 is a microscopic photograph (magnification ×10) showing the reactivity of monoclonal antibody 2H5 to sialyl Lewis X on vascular endothelial cells of pancreas, wherein the part (one example) indicated with an arrow is stained deep.

Fig. 20 is a microscopic photograph (magnification ×10) of a section of pancreas when mouse IgM (control) was used as a primary antibody instead of monoclonal antibody 2H5.

Fig. 21 is a microscopic photograph (magnification ×50) showing the reactivity of monoclonal antibody 2H5 to sialyl Lewis X on vascular endothelial cells of lung, wherein the part (one example) indicated with an arrow is stained deep.

Fig. 22 is a microscopic photograph (magnification ×50) of a section of lung when mouse IgM (control) was used as a primary antibody instead of monoclonal antibody 2H5.

Fig. 23 is a microscopic photograph (magnification ×50) showing the reactivity of monoclonal antibody 2H5 to sialyl Lewis X on vascular endothelial cells of blood vessel in mesentery, wherein the part (one example) indicated with an arrow is stained deep.

Fig. 24 is a microscopic photograph (magnification ×50) of a section of mesenteric blood vessel when mouse IgM (control) was used as a primary antibody instead of monoclonal antibody 2H5.

Fig. 25 is a microscopic photograph (magnification ×200) showing the reactivity of monoclonal antibody 2H5 to sialyl Lewis X on vascular endothelial cells of mesenteric blood vessel (artery), wherein the part (one example) indicated with an arrow is stained deep.

Fig. 26 is a microscopic photograph (magnification ×200) of a section of mesenteric blood vessel (artery) when mouse IgM (control) was used as a primary antibody instead of monoclonal antibody 2H5.

Fig. 27 is a microscopic photograph (magnification ×200) showing the reactivity of monoclonal antibody 2H5 to sialyl Lewis X on vascular endothelial cells of mesenteric blood vessel (vein), wherein the part (one example) indicated with an arrow is stained deep.

Fig. 28 is a microscopic photograph (magnification ×200) of a section of mesenteric blood vessel (vein) when mouse IgM (control) was used as a primary antibody instead of monoclonal antibody 2H5.

Fig. 29 is a graph showing an inhibitory effect on the adhesion of leukocytes to vascular endothelial cells, which is achieved by the administration of monoclonal antibody 2H5 in rat inflammatory model having an inflammation induced by histamine, wherein the vertical line is the number of leukocytes adhered onto vascular endothelial cells per 100 µm of post capillary venule of mesentery, the horizontal axis is time (minute), ○-○ is the value in the group administered with monoclonal antibody 2H5 and ●-● is the value in the control group.

Fig. 30 is a graph showing the inhibitory effect on the rolling of leukocytes on vascular endothelial cell membrane, which is achieved by the administration of monoclonal antibody 2H5 in rat inflammatory model having an inflammation induced by histamine, wherein the vertical line is the proportion (%) of leukocyte counts and the horizontal axis is the relative speed (%) of leukocytes rolling on vascular endothelial cell membrane.

Fig. 31 is a graph showing the leukocyte tissue infiltration-inhibitory effect by the administration of monoclonal antibody 2H5 in rat peritonitis model having an inflammation induced by thioglycolate, wherein the vertical line is the total number of leukocytes (cells) infiltrated into tissues.

Fig. 32 is a graph (solid line) showing the reactivity of monoclonal antibody 2H5 to human leukocytes (neutrophil) without treatment with neuraminidase, wherein the dotted line indicates the reactivity of mouse IgM (control antibody), the vertical line is relative cell counts and the horizontal axis is fluorescence intensity (logarithmic).

Fig. 33 is a graph (solid line) showing the reactivity of monoclonal antibody 2H5 to human leukocytes (neutrophil) which underwent treatment with neuraminidase, wherein the dotted line indicates the reactivity of mouse IgM (control antibody), the vertical line is relative cell counts and the horizontal axis is fluorescence intensity (logarithmic).

Fig. 34 is a graph (solid line) showing the reactivity of monoclonal antibody 2H5 to rat leukocytes (neutrophil) without treatment with neuraminidase, wherein the dotted line indicates the reactivity of mouse IgM (control antibody), the vertical line is relative cell counts and the horizontal axis is fluorescence intensity (logarithmic).

Fig. 35 is a graph (solid line) showing the reactivity of monoclonal antibody 2H5 to rat leukocytes (neutrophil) which underwent treatment with neuraminidase, wherein the dotted line indicates the reactivity of mouse IgM (control antibody), the vertical line is relative cell counts and the horizontal axis is fluorescence intensity (logarithmic).

Fig. 36 is a graph (solid line) showing the reactivity of monoclonal antibody 2H5 to mouse leukocytes (neutrophil) without treatment with neuraminidase, wherein the dotted line indicates the reactivity of mouse IgM (control antibody), the vertical line is relative cell counts and the horizontal axis is fluorescence intensity (logarithmic).

Fig. 37 is a graph (solid line) showing the reactivity of monoclonal antibody 2H5 to mouse leukocytes (neutrophil) which underwent treatment with neuraminidase, wherein the dotted line indicates the reactivity of mouse IgM (control antibody), the vertical line is relative cell counts and the horizontal axis is fluorescence intensity (logarithmic).

Fig. 38 is a photograph, in the place of a drawing, of a section of rat left ventricle after reperfusion after myocardial ischemia in the group administered with PBS.

Fig. 39 is a photograph, in the place of a drawing, of a section of rat left ventricle after reperfusion after myocardial ischemia in the group administered with monoclonal antibody 2H5.

Fig. 40 is a graph showing the effect of preventing injury (anti-inflammatory effect) by the pre-administration of monoclonal antibody 2H5 or rat IgM in rat model with reperfusion injury after myocardial ischemia, wherein the vertical line is a proportion (%) of necrosis area (infarotion) in the total area of the section of left ventricle.

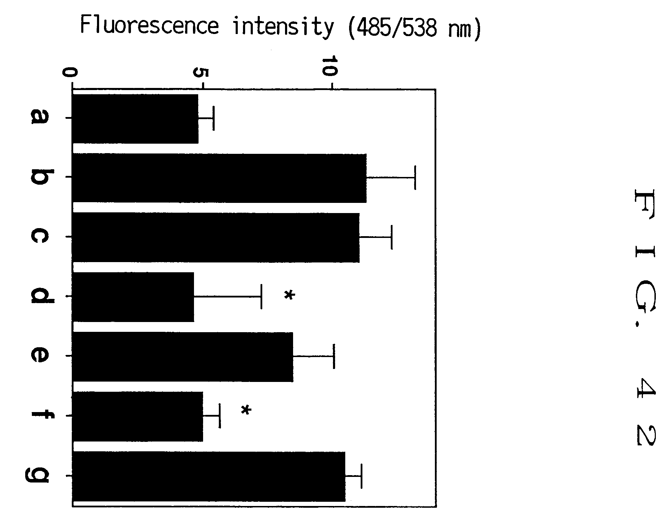

Fig. 41 is a graph showing the influence (effect) of monoclonal antibody 2H5 on the binding of E-selectin and HL-60 cells, wherein the vertical line is fluorescence intensity. In the horizontal axis, a shows the result of binding of HL-60 to untransformed COS cells and b-g show the result of binding of HL-60 to COS cells into which E-selectin gene had been introduced. In b-g, b shows the result when no antibody was added, c shows the result when 10 µg/ml of control mouse IgM was added, d shows the result when 10 µg/ml of monoclonal antibody 2H5 was added, e shows the result when 3 µg/ml of monoclonal antibody 2H5 was added, f shows the result when 5 µg/ml of P-selectin antibody was added, and g shows the result when 20 µg/ml of E-selectin antibody was added.

Fig. 42 is a graph showing the effect of monoclonal antibody 2H5 on the binding of P-selectin and HL-60 cells, wherein the vertical line is fluorescence intensity. In the horizontal axis, a shows the result of binding of HL-60 to untransformed COS cells, b-g show the result of binding of HL-60 to COS cells into which P-selectin gene had been introduced, b shows the result when no antibody was added, c shows the result when 10 µg/ml of control mouse IgM was added, d shows the result when 10 µg/ml of monoclonal antibody 2H5 was added, e shows the result when 3 µg/ml of monoclonal antibody 2H5 was added, f shows the result when 5 µg/ml of P-selectin antibody was added, and g shows the result when 20 µg/ml of E-selectin antibody was added.

### Detailed Description of the Invention

The present invention is described in detail by clarifying the meaning of the terms used in the present invention.

The "nonlymphoid tissues" in the present invention mean tissues other than peripheral lymphoid tissues which produce or preserve immunocompetent lymphocytes, such as lymph nodes, tonsil and spleen, and other than central lymphoid tissues such as thymus, which produce precursor cells which are origins of immunocompetent lymphocytes produced in said peripheral lymphoid tissues. Specific examples include tissues such as brain, trachea, lung, liver, heart, pancreas, intestine (small, large), mesentery, kidney, skin, nasal mucosa and joint, preferably lung, liver, heart, pancreas, skin, joint and kidney.

The "leukocytes" in the present invention include lymphocytes, neutrophils, eosinophils, basophils and monocytes, which are referred to generally as leukocytes.

The "sialyl Lewis X sugar chain" in the present invention means a sugar chain having a sialic acid residue and a Lewis X sugar chain in at least part of its structure. That is, said sugar chain need only have at least a sialic acid residue and a Lewis X sugar chain structure in its structure, and is free of particular limitations in terms of positional relationship between sialic acid residue and Lewis X sugar chain structure, structure of other sugar chains other than the Lewis X sugar chain, and the length thereof, and further may have either straight or branched sugar chain structure.

Specifically, according to the two dimensional expression by abbreviational nomenclature of glycolipids [see Kenzo Hirayama et al., Organic Chemistry, Biochemical Nomenclature (last volume), Nan-ko Do], there are included sugar chains having, in at least part of the structure, a sialic acid residue represented by NeuAc and a Lewis X sugar chain structure of Gal(β1-4)[Fuc(α1-3)]GlcNAc-, which are specifically exemplified by (1) straight or branched sugar chain under no particular limitation of sugar chain length, which has a sugar chain structure represented by NeuAc(α2-3)Gal(β1-4)[Fuc(α1-3)]GlcNAc-, (2) straight sugar chain under no particular limitation of sugar chain length, which has a sialic acid residue represented by NeuAc and a Lewis X sugar chain structure of Gal(β1-4)[Fuc(α1-3)]GlcNAc-, the both being located at a distance in the whole structure of the sugar chain and (3) branched sugar chain under no particular limitation of sugar chain length, which has a sialic acid residue represented by NeuAc and a Lewis X sugar chain structure of Gal(β1-4)[Fuc(α1-3)]GlcNAc-, the both being located in a direct bond or at a distance in the whole structure of the sugar chain.

The "sialyl Lewis X sugar chain" in the present invention includes various three dimensional streostructures that the above-mentioned two dimensional sialyl Lewis X sugar chain may form in the living body.

Moreover, the "sialyl Lewis X sugar chain" in the present invention is not particularly limited with regard to the mode of existence, as long as it is derived from vascular endothelial cell membranes of nonlymphoid tissues or cell membranes of leukocytes, and may be in the form of that expressed (present) on the cell membrane or in the form of that extracted/isolated from said cell membrane. It may be synthetic as long as it has the same structure as the sialyl Lewis X sugar chain derived from vascular endothelial cell membranes of nonlymphoid tissues or cell membranes of leukocytes. The sialyl Lewis X sugar chain expressed (present) on the cell membrane is not particularly limited by the mode of presence on the membrane, and may be present in any form on a protein or lipid on the cell membrane.

The "monoclonal antibody" in the present invention is a monoclonal antibody which has reactivity to at least a sialyl Lewis X sugar chain derived from vascular endothelial cell membranes of nonlymphoid tissues, and preferably has reactivity also to a sialyl Lewis X sugar chain derived from cell membranes of leukocytes. Examples thereof include monoclonal antibodies produced by hybridomas prepared according to Examples 1 and 2 to be mentioned below, preferably by hybridoma identified by international deposit No. FERM BP-4525.

The "monoclonal antibody" in the present invention may be a monoclonal antibody belonging to an immunoglobulin class of IgG, IgM, IgA, IgD or IgE, with preference given to a monoclonal antibody belonging to an IgG or IgM immunoglobulin class.

The "monoclonal antibody" in the present invention is not particularly limited in terms of production/obtainment method. For example, a hybridoma producing a monoclonal antibody is prepared according to a method generally used for producing an antibody, such as the method of Kohler and Milstein et al., [Nature, *256*, pp. 495-497 (1975)) or a modified method thereof, and then a monoclonal antibody can be obtained from said hydridoma. Specifically, there is exemplified a method comprising injecting, as an antigen, the above-mentioned sialyl Lewis X sugar chain or a substance containing said sugar chain subctaneously, intramuscularly or intraperitoneally to a mammal such as mouse, rat, guinea pig, hamster and rabbit, preferably mouse or rat and more preferably mouse, once to several times (immunization), and then fusing antibody-producing cells contained in spleen, lymph node, bone marrow or tonsil, preferably spleen, obtained from the immunized animal, and myeloma cell line (myeloma) of an animal (e.g., mammals such as mouse, rat, guinea pig, hamster, rabbit and human, preferably mouse, rat and human), which is preferably of the same species with the immunized animal, to give fused cells (hybridoma), which are cultured *in vitro* to give the antibody from the culture supernatant thereof. In addition, there is exemplified a method comprising culturing said hybridoma in ascites or serum (*in vivo*) of the same spices of the animal (e.g., mammals such as mouse, rat, guinea pig, hamster and rabbit, preferably mouse or rat and more preferably mouse) and obtaining the antibody from said ascites or serum.

The myeloma cell lines usable for cell fusion include, for example, mouse-derived myeloma P3/X63-AG8, P3/NSI/1-Ag-4-1, P3/X63-Ag8.U1, SP2/0-Ag14, FO and BW5147; rat-originated myeloma 210RCY3-Ag1.2.3.; human-originated myeloma U-266AR1, GM1500-6TG-A1-2, UC729-6, CEM-AGR, D1R11 and CEM-T15. The fused cell clones producing monoclonal antibody can be screened by culturing the fused cells in, for example, a microtiter plate, and by determining the reactivity to antigen of culture supernatant in the well in which cell growth is shown, by using, for example, an enzyme-antibody assay such as RIA and ELISA. In addition, the monoclonal antibody can be isolated and purified by subjecting the serum, ascites or culture supernatant obtained by the method mentioned above, which contains the monoclonal antibody, to saturated ammonium sulfate precipitation, euglobulin precipitation, ion-exchange chromatography (e.g., DEAE and DE52), or affinity column chromatography using anti-immunoglobulin column or protein A column.

While the "monoclonal antibodies" thus produced respectively have different sugar chains depending on the kind of mammals to be immunized, the "monoclonal antibody" of the present invention is not subject to any particular limitation imposed by such difference in the structure of the sugar chain, but encompasses monoclonal antibodies derived from any mammal.

The "monoclonal antibody" of the present invention encompasses "chimeric monoclonal antibody", "humanized monoclonal antibody" and "human monoclonal antibody" prepared using genetic engineering techniques.

One example of the method for producing these antibodies is specifically explained in the following. Note that the monoclonal antibody of the present invention is not particularly limited by such description.

### (i) Chimeric monoclonal antibody

This "chimeric monoclonal antibody" can be prepared by referring to Experimental Medicine, special issue, vol. 6, No. 10 (1988) and Japanese Patent Examined Publication No. 73280/1991. Specifically, for example, a C_{H} gene isolated from a second animal (the gene being a C gene encoding an H chain constant region) is operably linked so as to be expressed in the downstream of active V_{H} gene isolated from the DNA of antibody-producing cells of a first animal (the gene being a rearranged VDJ gene encoding an H-chain variable region) and a C_{L} gene isolated from the second animal (the gene being a C gene encoding an L chain constant region) is operably linked so as to be expressed in the downstream of active V_{L} gene isolated from the DNA of antibody-producing cells of the first animal (the gene being a rearranged VJ gene encoding an L-chain variable region). They are inserted into the same or different expression vector(s). Host cells are transformed with said expression vector(s), and the transformed cells are cultured, whereby chimeric monoclonal antibody is obtained.

In the case of mouse/human chimeric monoclonal antibody as the chimeric monoclonal antibody, the following method is employed.

A DNA is extracted from a mouse monoclonal antibody-producing hybridoma prepared by a conventional method, digested with a suitable restriction enzyme (e.g., EcoRI and HindIII), and subjected to electrophoresis using, for example, 0.7% agarose gel for Southern blot technique. The gel after running is stained with, for example, ethidium bromide. A photograph is taken and the location of the marker is marked. The gel is washed twice with water and immersed in a 0.25M HCl solution for 15 minutes. The gel is then immersed in a 0.4N NaOH solution for 10 minutes with gentle shaking. The gel is transferred to a filter by a conventional method, and 4 hours later, the filter is recovered and washed twice with 2×SSC. The filter is thoroughly dried and baked at 75°C for 3 hours. After the completion of the baking, the filter is placed in 0.1×SSC/0.1% SDS solution and treated at 65°C for 30 minutes. Then, the filter is immersed in 3× SSC/0.1% SDS solution. The obtained filter is placed in a plastic bag together with the prehybridization solution and treated at 65°C for 3-4 hours. Then, ³²P-labeled probe DNA and hybridization solution are placed therein and the reaction is carried out at 65°C for about 12 hours. After hybridization, the filter is washed at suitable salt concentration, reaction temperature and time (e.g., 2×SSC-0.1% SDS solution, room temperature, 10 min). The filter is placed in a plastic bag, added with a little amount of 2×SSC, sealed and subjected to autoradiography.

By the above-mentioned Southern blot technique, rearranged VDJ gene and VJ gene encoding H chain and L chain, respectively, of the objective monoclonal antibody are identified. The region including the identified DNA fragments are fractionated by sucrose density gradient centrifugation and integrated into phage vector (e.g., Charon 4A, Charon 28, λEMBL3 and λEMBL4). *Escherichia coli* (e.g., LE392 and NM539) is transformed with said phage vector to prepare a genomic library. The genomic library is subjected to plaque hybridization according to Benton · Davis method [Science, *196*, 180-182 (1977)] by using a suitable probe [e.g., H chain J gene and L chain (κ) J gene] to give positive clones individually including a rearranged VDJ gene or VJ gene. A restriction enzyme map of the obtained clones is depicted, and the nucleotide sequence is determined to confirm that a gene including the objective rearranged V_{H} (VDJ) gene or V_{L} (VJ) gene is obtained.

A human C_{H} gene and human C_{L} gene used for chimerization are separately isolated. For example, when a chimeric antibody with human IgG₁ is to be prepared, Cγ₁ gene as a C_{H} gene and Cκ gene as a C_{L} gene are isolated. These genes can be obtained by isolating from human genomic library by using, as probes, mouse Cγ₁ gene and mouse Cκ gene corresponding to human Cγ₁ gene and human Cκ gene, respectively, by utilizing the high homology between the nucleotide sequences of mouse immunoglobulin gene and human immunoglobulin gene.

Specifically, using 3 kb HindIII-BamHI fragment from clone Ig146 [Proc. Natl. Acad. Sci. USA, *75*, 4709-4713 (1987)] and 6.8 kb EcoRI fragment from clone MEP10 [Proc. Natl. Acad. Sci. USA, *78*, 474-478 (1981)] as probes, DNA fragment including human Cκ gene and carrying an enhancer region is isolated from HaeIII-AluI genomic library [Maniatis et al, Cell, *15*, 1157-1174 (1978)] of human λ Charon 4A. Human Cγ₁ gene is isolated by, for example, digesting human fetal hepatocyte DNA with HindIII, fractionating same by agarose gel electrophoresis, inserting a 5.9 kb band into λ 788, and using the above-mentioned probe.

Using mouse V_{H} gene and mouse V_{L} gene, and human C_{H} gene and human C_{L} gene thus isolated, human C_{H} gene is integrated into the downstream of mouse V_{H} gene, and human C_{L} gene is integrated into the downstream of mouse V_{L} gene in an expression vector such as pSV2gpt and pSV2neo by a conventional method using suitable restriction enzyme and DNA ligase, with consideration of promoter region, enhancer region and the like. In so doing, chimeric genes of mouse V_{H} gene/human C_{H} gene and mouse V_{L} gene/human C_{L} gene may be concomitantly placed in a single expression vector, or separately in different expression vectors.

The chimeric gene-inserted expression vector thus prepared is introduced into myeloma cells which do not produce antibody by themselves, such as P3X63 · Ag8 · 653 cell and SP210 cell, by protoplast fusion, DEAE-dextran method, calcium phosphate method or electroporation method. The transformed cells are selected according to the culture in a medium containing a drug, which corresponds to a drug resistant gene introduced into the expression vector, whereby the objective chimeric monoclonal antibody-producing cells are obtained.

The objective chimeric monoclonal antibody is obtained from the supernatant of the culture of the antibody-producing cells thus selected.

### (ii) Humanized monoclonal antibody

This "humanized monoclonal antibody" is a monoclonal antibody (CDR-grafted monoclonal antibody) wherein the entire region of CDR region (complementality-determining residue) consisting of three regions (CDR1, CDR2, CDR3), or the entire region except a part thereof has been replaced for the corresponding region of human monoclonal antibody. These regions are present in hypervariable region in the variable region of monoclonal antibody of non-human animals, and are directly bound complementarily with antigen. Those can be prepared by genetic engineering technique by reference to, for example, Japanese Patent Application under PCT laid-open under Kohyo No. 506458/1992 and Japanese Patent Unexamined Publication No. 296890/1987, as in the case of the above-mentioned chimeric monoclonal antibody.

That is, at least one CDR gene of mouse H chain and at least one CDR gene of mouse L chain corresponding to said H chain CDR gene are isolated from a specific mouse monoclonal antibody-producing cell such as the above-mentioned hybridoma, and human H chain gene encoding the entire region except human H chain CDR corresponding to the above-mentioned mouse H chain CDR, and human L chain gene encoding the entire region except human L chain CDR corresponding to mouse L chain CDR, are isolated from human immunoglobulin gene. The isolated mouse H chain CDR gene and human H chain gene are introduced into a suitable expression vector to allow expression, and similarly, mouse L chain CDR gene and human L chain gene are introduced into another suitable expression vector to allow expression. Alternatively, mouse H chain CDR gene/human H chain gene and mouse L chain CDR gene/human L chain gene may be introduced into the same expression vector to allow expression. Host cells are transformed with the expression vector thus prepared to give humanized antibody-producing transformant, and then the objective humanized monoclonal antibody is obtained from the culture supernatant of the transformant.

### (iii) Human monoclonal antibody

"Human monoclonal antibody" can be obtained in the same manner as above by immunizing a specific antigen to transgenic mouse prepared by, for example, integrating human immunoglobulin gene into the genome of the mouse. Compared to the above-mentioned chimeric monoclonal antibody and humanized monoclonal antibody, the entire region is originated from human immunoglobulin gene.

The transgenic mouse which produces this human type monoclonal antibody can be prepared, for example, according to a method described in Nature Genetics, vol. 7, pp. 13-21 (1994) and Japanese Patent Application under PCT laid-open under Kohyo No. 504365/1992, or Nature, vol. 368, pp. 856-859 (1994) and Japanese Patent Application under PCT laid-open under Kohyo No. 500233/1994.

The monoclonal antibodies of the present invention described above in detail can suppress or inhibit a reaction in at least one step in a series of reactions mediating inflammatory responses generally summarized in the following ①-⑤. That is, ① migration to vascular endothelial cell membrane, ② initial adhesion onto vascular endothelial cell membrane via selectins, ③ rolling on the vascular endothelial cell membrane, ④ strong adhesion onto vascular endothelial cell membrane via integrins after said rolling, and ⑤ infiltration into extravascular tissues after said strong adhesion, of leukocytes (lymphocytes, neutrophils, eosinophils, basophils and monocytes) usually flowing in the blood in blood vessels at a high speed.

The monoclonal antibody in the present invention is clinically useful as a leukocyte rolling inhibitor, a leukocyte adhesion inhibitor or an inhibitor of infiltration of leukocytes into tissues. It is also useful as an anti-inflammatory agent for the prophylaxis or treatment of inflammation caused via the above-mentioned steps.

The "leukocyte rolling inhibitor" of the present invention is a preparation characteristically comprising the above-mentioned monoclonal antibody, namely, the monoclonal antibody having reactivity to sialyl Lewis X sugar chains derived from vascular endothelial cell membranes of nonlymphoid tissues, preferably further having, in addition to said reactivity, reactivity to sialyl Lewis X sugar chains derived from leukocyte cell membranes. This leukocyte rolling inhibitor reduces the degree of rolling or inhibits the rolling of leukocytes on vascular endothelial cell membranes of nonlymphoid tissues, which occurs (or has occurred) in the above-mentioned step ③. Specifically, the action mechanism of this preparation is considered to be based on suppression and inhibition of rolling by increasing the speed of rolling of leukocytes, which are rolling while adhering to vascular endothelial cells via binding of sialyl Lewis X sugar chain and selectin, thereby placing them again in the blood stream.

The "leukocyte adhesion inhibitor" of the present invention is a preparation characteristically comprising the above-mentioned monoclonal antibody, i.e., monoclonal antibody having reactivity to sialyl Lewis X sugar chains derived from vascular endothelial cell membranes of nonlymphoid tissues, preferably that further having, in addition to said reactivity, reactivity to sialyl Lewis X sugar chain derived from leukocytes. This preparation reduces the degree of adhesion or inhibits the adhesion of leukocytes onto vascular endothelial cell membranes of nonlymphoid tissues, which occurs (or has occurred) in the above-mentioned steps ①-④.

The "inhibitor of infiltration of leukocytes into tissues" of the present invention is a preparation characteristically comprising the above-mentioned monoclonal antibody, i.e., monoclonal antibody having reactivity to sialyl Lewis X sugar chains derived from vascular endothelial cell membranes of nonlymphoid tissues, preferably that further having, in addition to said reactivity, reactivity to sialyl Lewis X sugar chain derived from leukocytes. This preparation reduces the degree of exudation or inhibits the exudation of leukocytes in the blood stream (intravascular) to the outside of blood vessels, namely, exudation (infiltration) into tissues, which occurs (or has occurred) in the above-mentioned step ⑤.

The "anti-inflammatory agent" of the present invention is a preparation characteristically comprising the above-mentioned monoclonal antibody, i.e., monoclonal antibody having reactivity to sialyl Lewis X sugar chains derived from vascular endothelial cell membranes of nonlymphoid tissues, preferably that further having, in addition to said reactivity, reactivity to sialyl Lewis X sugar chains derived from leukocytes. This preparation prevents or suppresses/inhibits (treats) inflammatory responses in tissues based on the suppression/inhibition of the reaction which occurs in at least one step of the above-mentioned steps ①-⑤.

The "inflammation" in the present invention, namely, the inflammation to be the target of the anti-inflammatory agent of the present invention includes basic pathological local reactions comprising a kinetic combination of cytologic and histological responses of blood vessels and adjacent tissues, which are caused by infiltration of leukocytes from the blood stream to extravascular tissues via rolling on vascular endothelial cell membrane and adhesion thereto, which are found in damages or dysfunction of the living tissues caused by various factors not limited to internal factors and external factors of bacterial infection, trauma, physical stimulation such as heat, cold, radiation, electricity and the like, or chemical substance.

Generally, inflammation can be roughly grouped into chronic and acute ones depending on the expression rate and the degree of progression. In general terms, chronic inflammation means that which expresses comparatively slowly or gradually to the degree that its absence or presence of expression is unclear, and which is maintained over several weeks to several years wherein the time of disappearance is unknown. Acute inflammation means that which expresses comparatively rapidly and progresses fast, wherein its disappearance is comparatively clear. The acute inflammation in the present invention means those in the sense commonly used as mentioned above.

Examples of the inflammation in the present invention include inflammations in the tissues such as brain, trachea, blood vessel, lung, liver, heart, pancreas, intestine, mesentery, kidney, skin, nasal mucosa and joint, which are specifically exemplified by cerebritis, bronchitis, angitis, pneumonia, hepatitis, myocarditis, pancreatitis, enteritis, peritonitis, dermatitis, nephritis and arthritis (e.g., rheumatoid arthritis), as well as inflammations associated with reperfusion injury after ischemia, immune rejection after transplantation, burns or multiple organ failure.

As the inflammations targeted by the anti-inflammatory agent of the present invention, acute inflammations are specifically exemplified, which include acute inflammation in tissues such as brain, trachea, blood vessel, lung, liver, heart, pancreas, intestine, mesentery, kidney, skin, nasal mucosa and joint, specifically, acute hepatitis, acute pneumonia and acute inflammations associated with reperfusion injury after ischemia, immune rejection after transplantation, burns or multiple organ failure.

The leukocyte rolling inhibitor, the leukocyte adhesion inhibitor, the inhibitor of infiltration of leukocytes into tissues, and the anti-inflammatory agent of the present invention can be prepared into an injectable form by, for example, dissolving or suspending the above-mentioned monoclonal antibody in a nontoxic and pharmaceutically acceptable carrier, such as physiological saline, to a concentration of from 0.1 µg antibody/ml carrier to 1 mg antibody/ml carrier. The injection thus prepared can be administered to mammals in need of the treatment in a dose of 1 µg-100 mg, preferably 50 µg-50 mg, per 1 kg body weight, once to several times a day. The administration mode is exemplified by the administration routes suitable for medical treatments, such as intravenous injection, subcutaneous injection, intracutaneous injection, intramuscular injection and intraperitoneal injection, with preference given to intravenous injection.

The leukocyte rolling-inhibitory effect of the leukocyte rolling inhibitor of the present invention can be confirmed by, for example, measuring rotational migration speed (rolling speed) of the leukocytes in the blood stream upon administration of the monoclonal antibody of the present invention, according to the method of Tamatani et al., [Eur. J. Immunol., vol. 23, pp. 2181-2188 (1993)].

The leukocyte adhesion-inhibitory effect of the leukocyte adhesion inhibitor of the present invention can be confirmed by, for example, measuring the inhibition ratio of the adhesion of leukocytes to vascular endothelial cell membrane per unit area and unit time upon administration of the monoclonal antibody of the present invention, according to the method of Suematsu et al., [Am. J. Physiol., vol. 264, pp. H881-H891 (1993)].

The leukocyte tissue infiltration-inhibitory effect of the leukocyte tissue infiltration inhibitor of the present invention can be confirmed by, for example, counting the infiltrated leukocytes in abdominal cavity upon administration of the monoclonal antibody of the present invention according to the method of Tanya N. Mayadas et al., [Cell, vol. 74, pp. 541-554 (1993)].

The anti-inflammatory effects of the anti-inflammatory agent of the present invention on specific inflammation can be confirmed by, for example, according to the method of Mulligan et al., [J. Clin. Invest., vol. 91, pp. 577-587 (1993)].

The monoclonal antibody prepared by the present inventors has reactivity to not only sialyl Lewis X sugar chains derived from the cell membrane of leukocytes, which being one of the *in vivo* ligands for E-selectin and P-selectin, but also to sialyl Lewis X sugar chains derived from vascular endothelial cell membranes. The use of the instant monoclonal antibody enables inhibition of rolling and adhesion of leukocytes on vascular endothelial cell membranes, and infiltration of leukocytes to extravascular tissues, all of which are mediated by sialyl Lewis X sugar chains on vascular endothelial cell membranes, or sialyl Lewis X sugar chains on both of vascular endothelial cell membranes and leukocyte cell membranes. Accordingly, the present invention can provide clinically useful leukocyte rolling inhibitor, leukocyte adhesion inhibitor and leukocyte tissue infiltration inhibitor.

The monoclonal antibody of the present invention has multiplicate anti-inflammatory effects, which are the combined effects of those of respective monoclonal antibodies to the above-mentioned selectin proteins (E-, P- and L-selectins). Consequently, the present invention can provide a potent anti-inflammatory agent based on a new action mechanism heretofore unknown, which is based on the inhibition of the very early stages of inflammatory responses in local tissue, namely, rolling and adhesion of leukocytes on vascular endothelial cells, as well as infiltration of leukocytes into tissues.

The present invention is described in more detail in the following by way of illustrative Examples. It is needless to say that the present invention is not limited to the embodiments described in the following.

### Example 1

### Preparation of antigen (glycolipid having sialyl Lewis X sugar chain)

Human colon cancer cells LS174T (ATCC CL188) were cultured in Dulbecco MEM medium containing 10% fetal calf serum. Said tumors were xenografted to nude mice [see Kannagi et al., Handbook of Experimental Immunology, vol. 1, pp. 9.1-9.39 (1986)] and grown. The tumor tissue was excised and extracted with a mixed solution of isopropanol/hexane/water (55:20:25, v/v/v), followed by recovery of total glycolipid. The glycolipid thus obtained was partitioned by Folch's partition method, and the glycolipid fraction in the upper layer was applied to DEAE-Sephadex A-25 column chromatography (Pharmacia). In said chromatography, neutral glycolipids were eluted with an eluent of chloroform/methanol/water (30:60:8, v/v/v) (fraction A) and gangliosides were eluted with an eluent of chloroform/methanol /0.8 N sodium acetate (30:60:8, v/v/v) (fraction B). The gangliosides in the fraction B were further separated by thin layer chromatography [hereinafter TLC, used were HPTLC plate : Si-HPF plate (J.I. Baker Chemical) and ¹²⁵I-protein A (Dupont)] using a solvent system of chloroform/methanol/2% dichlorocalcium (60:35:8, v/v/v) and immunostained [see Kannagi et al., Handbook of Experimental Immunology, vol. 4, pp. 117.1-117.21 (1986)] with FH6 monoclonal antibody obtained from Biomembrane Institute. Glycolipids having 10 or more sugar residues which showed cross reactivity to FH6 monoclonal antibody were recovered.

By the reactivity testing to anti-I antibody (IgM-Ma Puget Sound Blood Bank, Seattle), the recovered glycolipid was confirmed to be sialyl Lewis X active, and have branched core structure, and longer and more complicated carbonhydrate structure than known short chain sialyl Lewis X active glycolipids.

Then, said sialyl Lewis X active glycolipids were absorbed to *Salmonella minnesota* R595 strain (ATCC 49284).

### Example 2

### Preparation of monoclonal antibody

The following antibody-producing hybridomas were prepared by refe rence to the method of Kohler et al., [Omori et al., Blood, vol. 81, pp. 101-111 (1993)] and monoclonal antibody was prepared by reference to the method of Kannagi et al., [Handbook of Experimental Immunology, vol. 4, pp. 117.1-117.21 (1986)].

The *Salmonella minnesota* R595 strain (ATCC 49284) which absorbed sialyl Lewis X active glycolipid prepared in Example 1 was used as an immunization antigen. The antigen was intraperitoneally administered to BALB/c mice at such intervals and amounts as day 0 (7 µg), day 7 (15 µg), day 14 (23 µg) and day 28 (23 µg). At 3 days after the final immunization, the mice underwent celiotomy, whereby spleen cells were harvested and fused with mouse myeloma cells P3/X63-Ag8U1 (P3U1) (ATCC CRL1597) by a conventional method.

The culture supernatants of respective hybridomas obtained were applied to solid phase enzyme-immunoassay by a conventional method using the sialyl Lewis X active glycolipid prepared in Example 1 as an immunization antigen, and hybridoma clone having reactivity to said sialyl Lewis X active glycolipid was obtained, which was named as hybridoma clone 2H5. This hybridoma was deposited at National Institute of Bioscience and Human-Technology Agency of Industrial Science and Technology under the international deposit No. FERM BP-4525.

The hybridoma clone 2H5 was cultured in serum-free medium (ASF104, manufactured by Ajinomoto), and the culture supernatant was recovered, which was subjected to centrifugal separation (10,000 rpm, 10 min). The centrifugation supernatant was recovered and the monoclonal antibody produced by the hybridoma clone 2H5 (which antibody was named as monoclonal antibody 2H5) was purified therefrom using an IgM purification kit (manufactured by Pierce).

Using a mouse monoclonal antibody isotype identification kit (Amersham), the immunoglobulin class of said monoclonal antibody 2H5 was identified to be IgM.

### Example 3

### Reactivity of monoclonal antibody 2H5 to sialyl Lewis X sugar chain

The reactivity of monoclonal antibody 2H5 prepared in Example 2 to sialylated sugar chain antigen (using sialyl Lewis X sugar chain and sialyl Lewis **a** sugar chain) was examined by ELISA (enzyme-linked immunosorbent assay) by using a 96 well microtiter plate by reference to the method of Hakomori et al. [Handbook of Experimental Immunology, vol. 1, pp. 9.1-9.39 (1986)].

As the sialyl Lewis X sugar chain antigens, synthetic NeuAc(α2-3)Gal(β1-4)[Fuc(α1-3)]GlcNAc(β1-3)Gal(β1-4)Glc(β1-1)Cer was used and as the sialyl Lewis **a** sugar chain, synthetic NeuAc(α2-3)Gal(β1-3)[Fuc(α1-4)]GlcNAc(β1-3)Gal(β1-4)Glc(β1-1)Cer was used. As an enzyme-labeled secondary antibody, peroxidase-labeled goat anti-mouse IgM antibody (Cappel) was used for the both.

For comparison, SNH-3 (IgM, obtained from Biomembrane Institute), which is a known anti-sialyl Lewis X monoclonal antibody, and 2D3 [IgM, see Biochem. Biophys. Res. Commun., *179*, 713-716 (1991) and Cancer Res., *53*, 354-361 (1993)], which is a known anti-sialyl Lewis **a** monoclonal antibody, were used instead of monoclonal antibody 2H5.

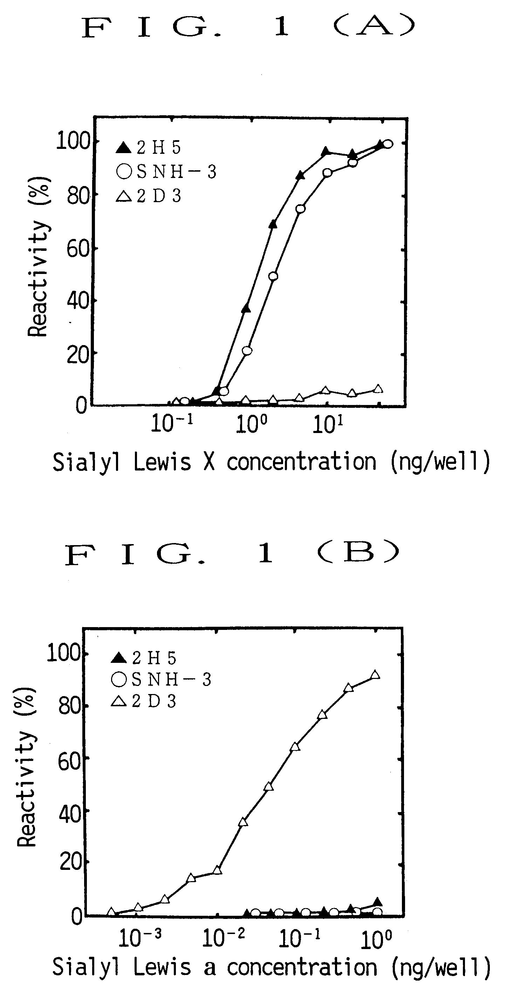

The results are shown in Fig. 1, A and B.

From these results, it was proved that the monoclonal antibody 2H5 of the present invention had a strong reactivity to sialyl Lewis X sugar chain antigen alone.

### Example 4

### Reactivity of monoclonal antibody 2H5 to sialyl Lewis X sugar chain on cell membrane of lymph node stroma

The reactivity of monoclonal antibody 2H5 to sialyl Lewis X sugar chain on membrane protein was examined by Western blotting analysis on sodium dodecylsulfate-polyacrylamide gel electrophoresis (hereinafter referred to as SDS-PAGE) by using 10% polyacrylamide gel, with reference to the method of Laemmli, U.K. et al [Nature, vol. 227, pp. 680-685 (1970)].

For immunoblotting, solubilized glycoproteins on cell membrane of human peripheral lymph node stroma, obtained by the use of sodium dodecylsulfate (hereinafter SDS, protein solubilizing agent) by reference to the method of Towbin et al., [Proc. Natl. Acad. Sci. U.S.A., vol. 76, pp. 4350-4354 (1979)], were applied to SDS-PAGE and then transferred onto a nitrocellulose membrane (Schleicher & Schnell). This nitrocellulose membrane was reacted with monoclonal antibody 2H5 (IgM), then with peroxidase-labeled goat anti-mouse IgM antibody (Zymed) as an enzyme-labeled secondary antibody, and allowed to develop color with 3,3'-diamino-benzidine. As a control, SNH-3 monoclonal antibody (IgM, obtained from Biomembrane Institute) was used instead of monoclonal antibody 2H5 and examined in the same manner.

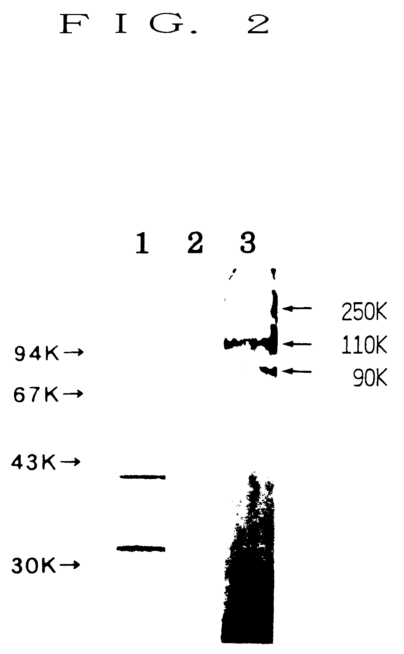

The results are shown in Fig. 2. From these results, it was proved that the monoclonal antibody 2H5 had reactivity to ca. 250 kD, 110 kD and 90 kD glycoproteins on cell membrane of human peripheral lymph node stroma, and that the monoclonal antibody 2H5 of the present invention had reactivity to sialyl Lewis X derived from cell membrane of lymph node stroma.

### Example 5

### Reactivity of monoclonal antibody 2H5 to sialyl Lewis X sugar chain on rat leukocyte cell membrane

The reactivity of monoclonal antibody 2H5 to sialyl Lewis X sugar chain on rat leukocyte cell membrane was examined by a conventional method according to fluorochrome labeling antibody method.

The peripheral blood of Wistar rat (WKY rat) was obtained from heart blood. Erythrocytes were hemolyzed with a non-isotonic solution to give leukocytes. The leukocytes (1×10⁶ cells) were reacted with monoclonal antibody 2H5, then with FITC (fluoroscein isothiocyanate)-labeled goat anti-mouse IgM antibody (Cappel), and fluorescence intensity of neutrophile fraction was determined by flow cytometer (Coulter).

Using, as a control, isotype matched mouse IgM (Sigma) instead of monoclonal antibody 2H5, a similar determination was performed.

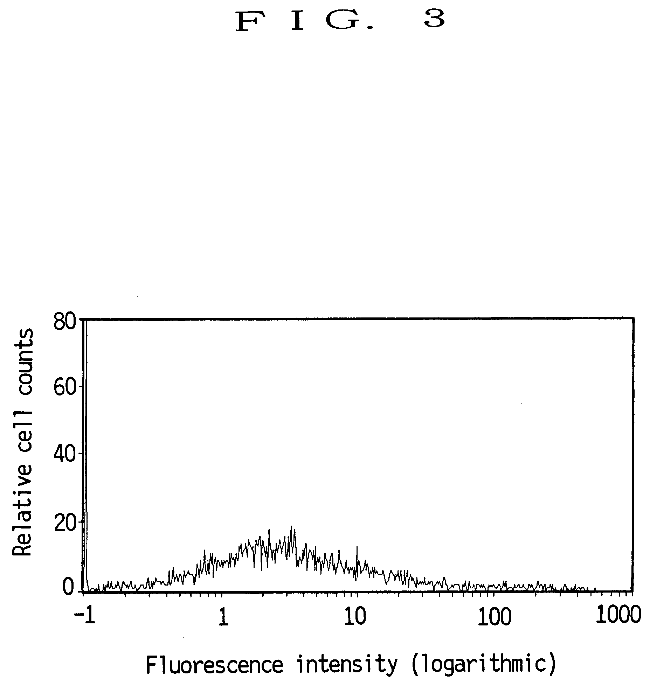

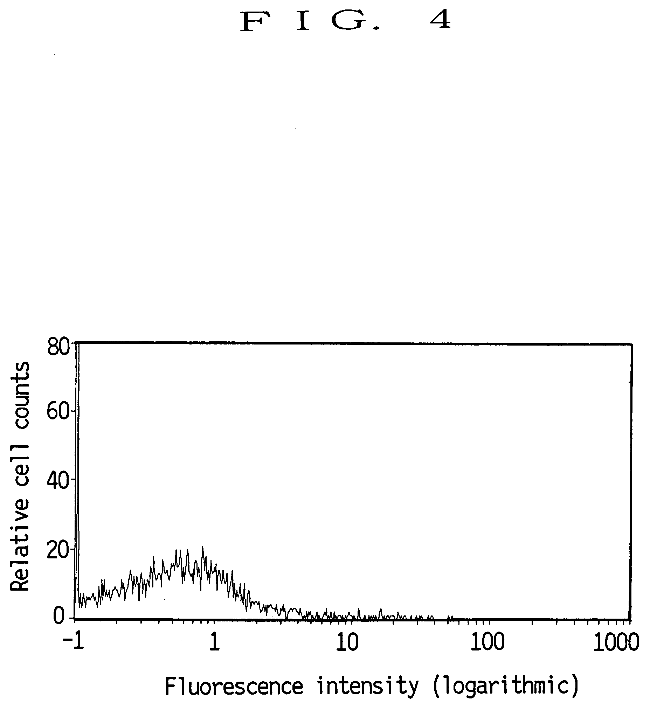

The results are shown in Figs. 3 and 4. From these results, it was proved that the monoclonal antibody 2H5 of the present invention had strong reactivity to sialyl Lewis X sugar chain on leukocyte cell membrane.

### Example 6

### Reactivity of monoclonal antibody 2H5 to sialyl Lewis X sugar chain on human leukocyte cell membrane

In the same manner as in Example 5, the reactivity of monoclonal antibody 2H5 to sialyl Lewis X sugar chain on human leukocyte cell membrane was examined.

Peripheral blood was obtained from healthy human, and erythrocytes were hemolyzed with a non-isotonic solution to give leukocytes. The leukocytes (1×10⁶ cells) were reacted with monoclonal antibody 2H5, then with FITC (fluoroscein isothiocyanate)-labeled goat anti-mouse IgM antibody (Cappel), and fluorescence intensities of monocyte fraction and granulocyte fraction were determined respectively by flow cytometer (Becton Dickinson).

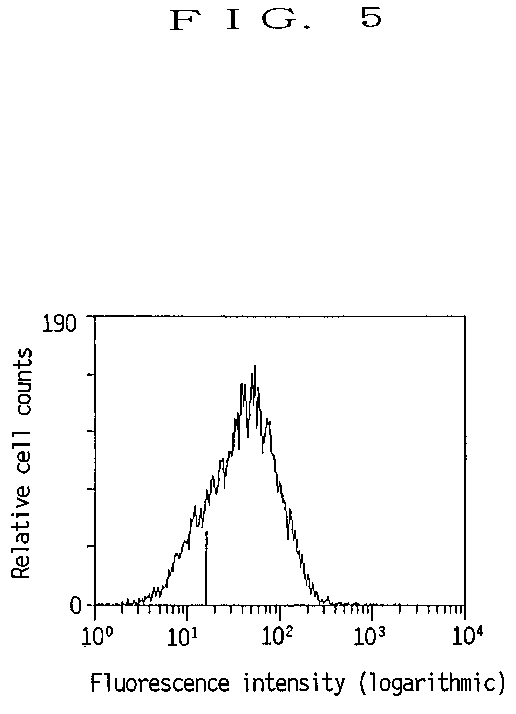

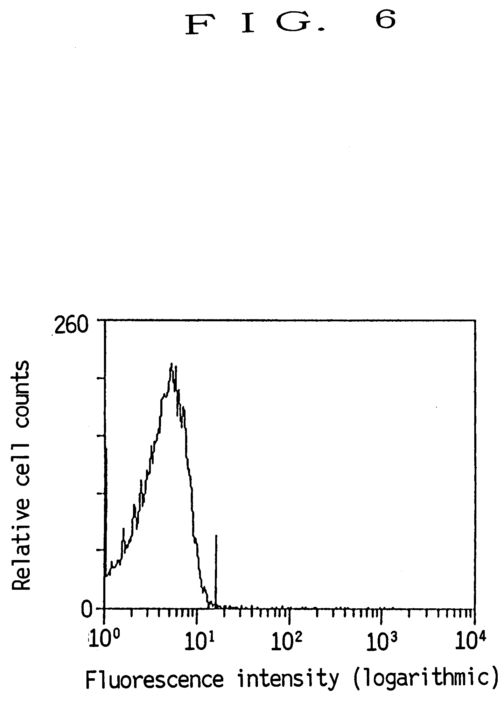

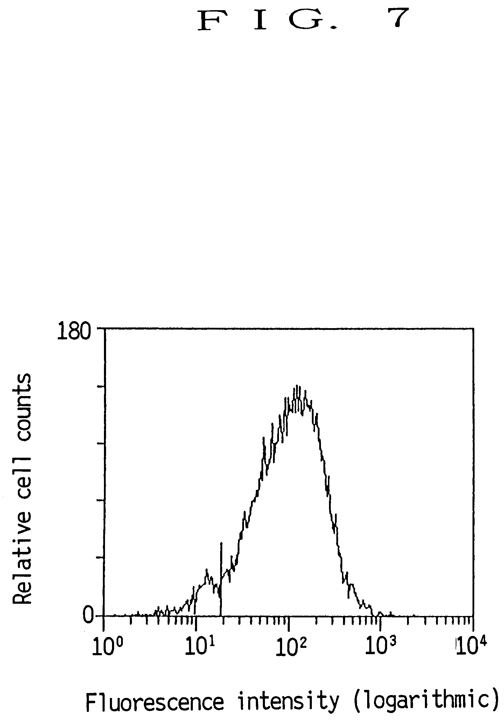

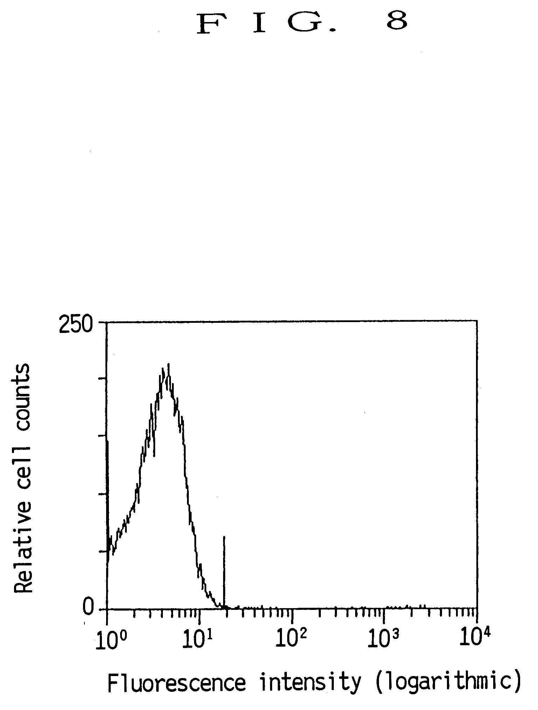

Using, as a control, isotype matched mouse IgM (Sigma) instead of monoclonal antibody 2H5, a similar determination was performed. The results are shown in Figs. 5 to 8. It was proved that the monoclonal antibody 2H5 of the present invention had strong reactivity to sialyl Lewis X sugar chain on human leukocyte cell membrane.

### Example 7

### Reactivity of monoclonal antibody 2H5 to sialyl Lewis X sugar chain on vascular endothelial cell membranes of various tissues

Peripheral lymph node, mesenteric lymph node, liver, kidney, spleen, lung and mesenteric blood vessels were harvested from Wistar rat (WKY rat) by surgery, and frozen sections of each tissue were prepared by a conventional method. The respective sections thus prepared were stained using a Vectastain Elite ABC kit (Funakoshi Corp.) as mentioned below.

First, frozen sections of each tissue were fixed with acetone for 1-2 minutes, and after drying, infiltrated with diluted horse serum [PBS (10 ml)/serum (150 µl)]. After washing with PBS, the monoclonal antibody 2H5 (10 µg/ml) prepared in Example 2 was added as a primary antibody, and the tissues were stood for 30 minutes. Then, after washing with PBS, 100 µl of biotinylated secondary antibody solution [PBS (5 ml)/rat serum (150 µl)/biotinylated anti-mouse secondary antibody (50 µl)] was added, which was followed by standing for 30 minutes. The tissues were stood still for 10 minutes in a solution of 3% hydrogen peroxide dissolved in methanol, washed with PBS, and added with 100 µl of avidin-peroxidase solution [PBS (5 ml) /peroxidase-labeled avidin DH (100 µl)/biotinylated hydrogen peroxide H (100 µl)], which was followed by standing for 30 minutes. After washing with PBS, DAB (diamino-benzidine tetrahydrochloride) solution [water (5 ml)/buffer solution (100 µl)/DAB solution (200 µl)/hydrogen peroxide solution (100 µl)/nickel solution (100 µl)] was added and the mixture was stood for 2-10 minutes. After washing with cold water for 5 minutes, the sections were subjected to Giemsa staining and then to mounting. The sections obtained by staining in the same manner as above by using mouse IgM (Chemcon) as a primary antibody instead of monoclonal antibody 2H5, were used as controls.

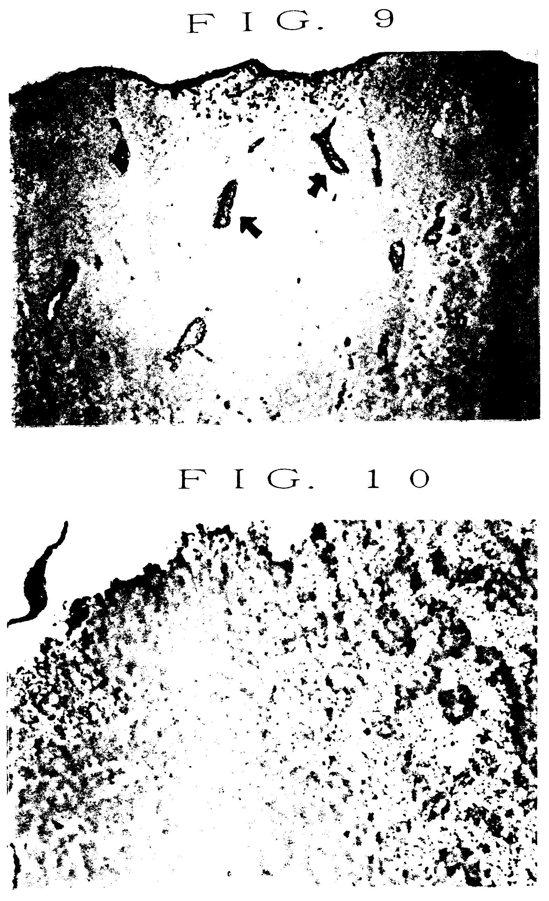

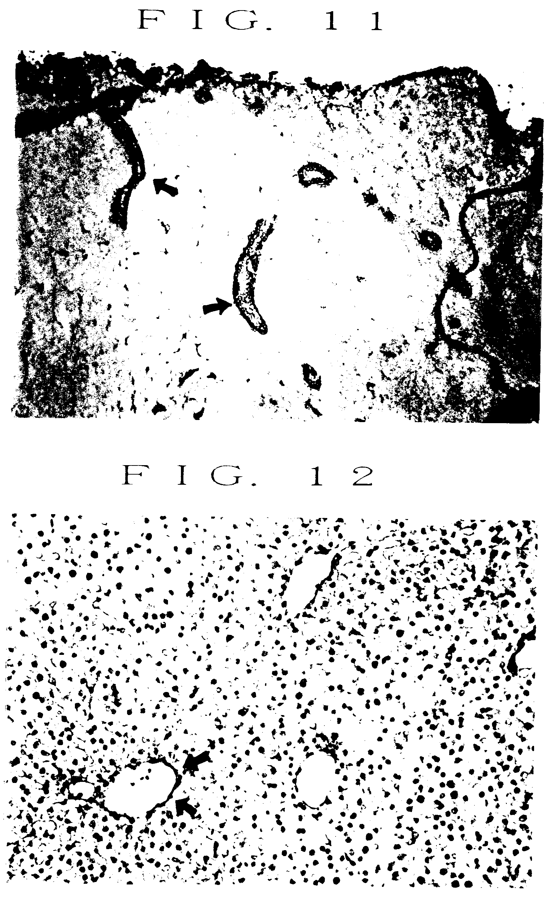

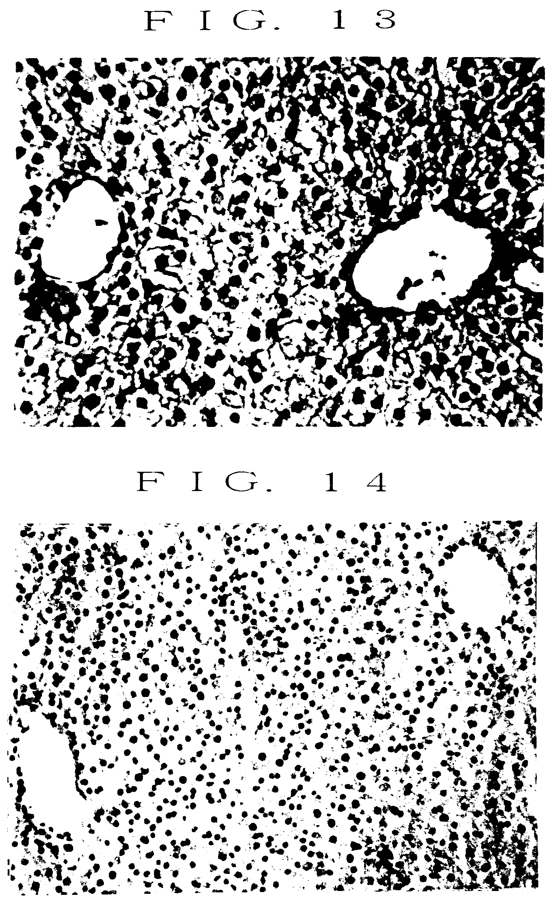

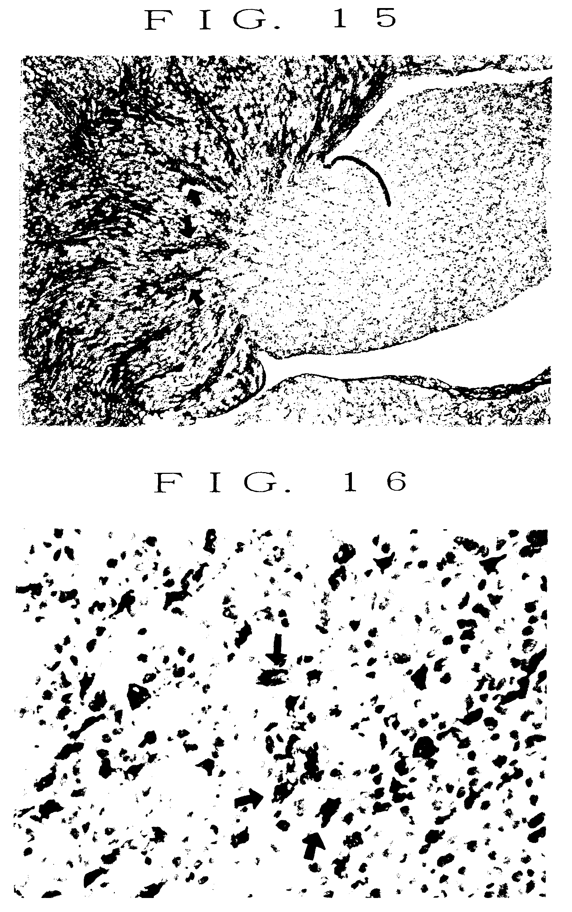

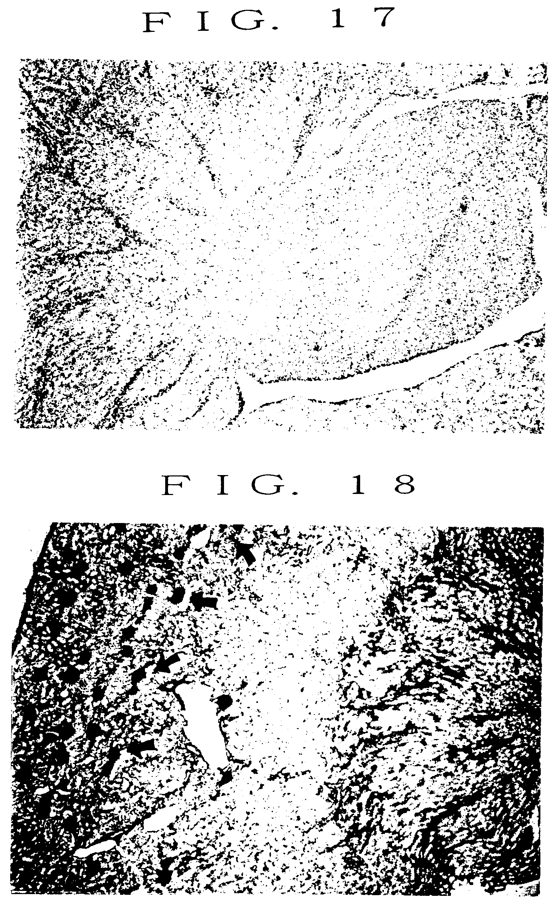

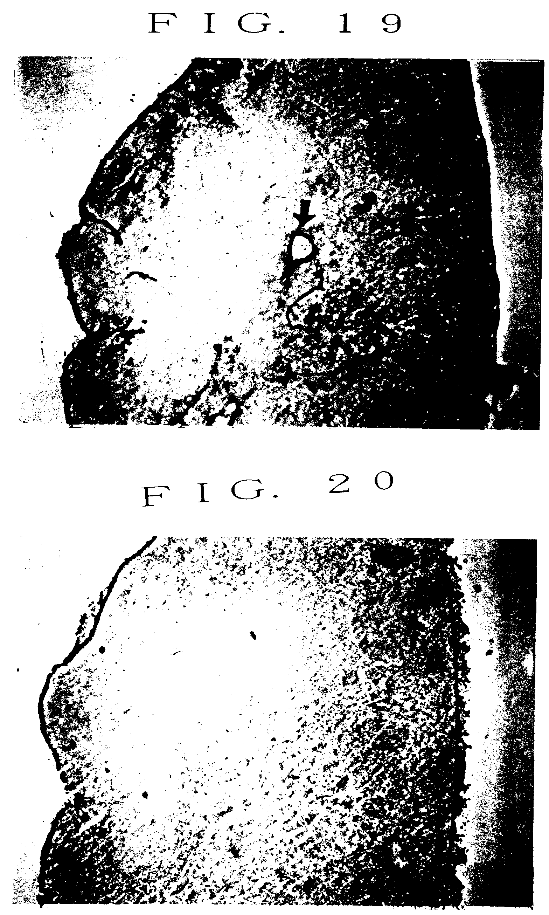

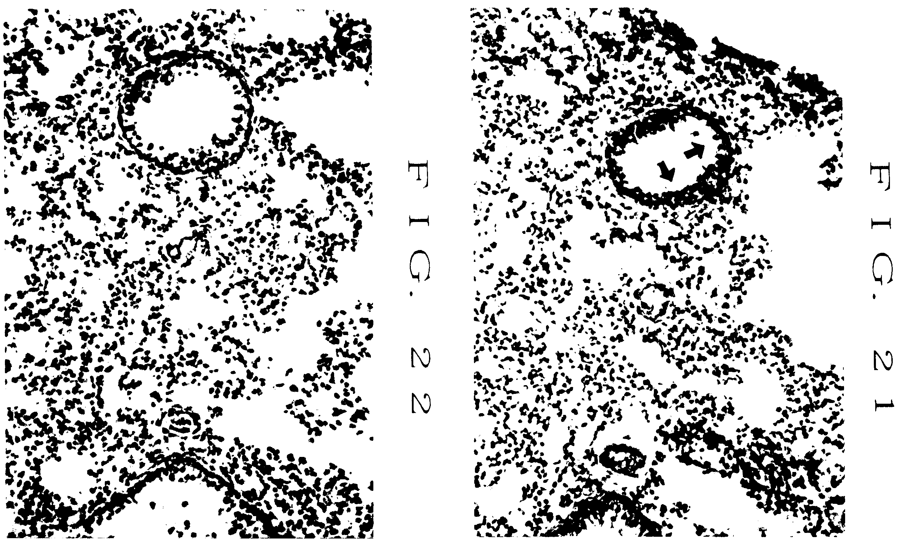

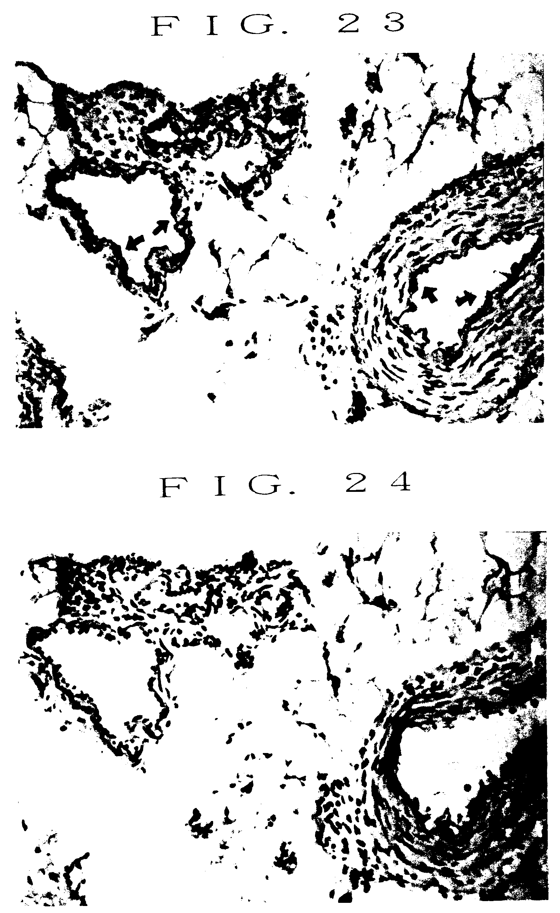

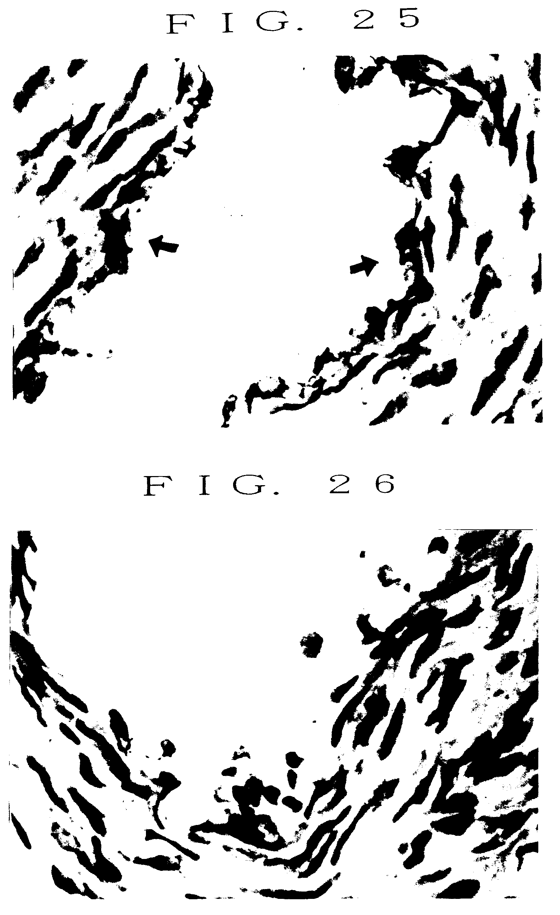

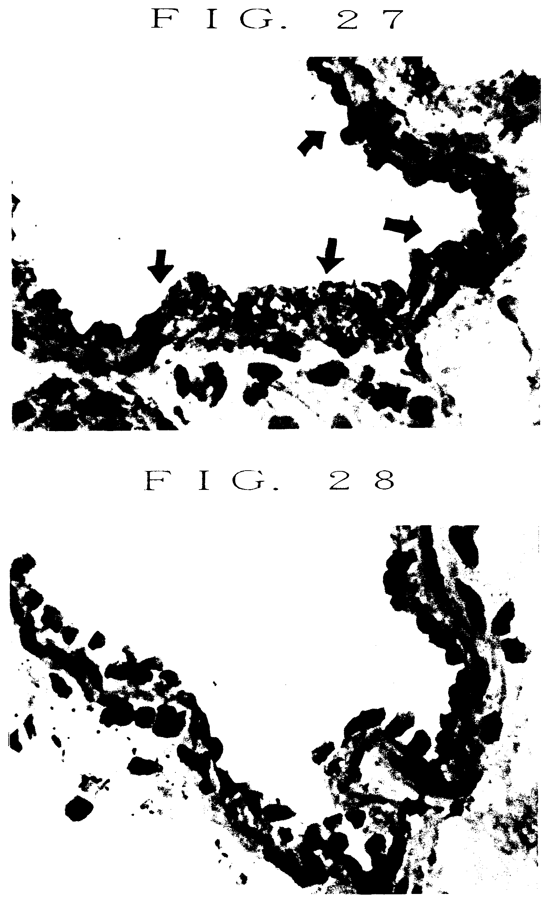

The stained and mounted tissue sections were examined by microscopy at magnifications of from ×10 to ×200, the results of which are shown in Figs. 9-28 (microscopic photographs).

The vascular endothelial cells of peripheral lymph node, mesenteric lymph node, liver, kidney, spleen, lung and mesenteric blood vessels were stained deep, and it was proved that the monoclonal antibody 2H5 of the present invention had strong reactivity not only to sialyl Lewis X on lymphoid tissues such as peripheral lymph node, mesenteric lymph node and spleen, but also sialyl Lewis X on nonlymphoid tissues such as liver, kidney, lung and mesenteric blood vessels.

### Example 8

### Inhibitory effect of monoclonal antibody 2H5 on adhesion of leukocytes in rat models with inflammation induced by histamine

Using rat models with inflammation induced by histamine, inhibitory effect of monoclonal antibody 2H5 on adhesion of leukocytes to vascular endothelial cell membrane was examined.

WKYs rats (12 weeks old, male, 290-320 g, 5 rats) were anesthetized by an intramuscular injection of sodium pentobarbital (40 mg/kg) and stabilized for 30 minutes. The abdomen was incised by surgery and mesentery was pulled out. After perfusion under 95% nitrogen/5% oxygen concentrations, the mesentery was placed under orthostereobiomicroscopy and the number of leukocytes which adhered to vascular endothelial cells of post capillary venule of mesentery (30-40 µm) was counted for 15 minutes through videotaping. Then, monoclonal antibody 2H5 [monoclonal antibody 2H5 (600 µl/diluted with physiological saline (900 µl)] was intravenously administered at a concentration of 2 mg/kg from femoral vein over 15 minutes. At 15 minutes after the termination of administration of monoclonal antibody 2H5, histamine (10 µM) solution was added to the mesentery. At the termination of histamin administration (0 min), 10 minutes later, 20 minutes later, 30 minutes later and 40 minutes later, the number of leukocytes which adhered to vascular endothelial cells of post capillary venule of mesentery (30-40 µm) under the microscopy was counted in the same manner as above.

As a control, the group of 5 rats administered with histamine without pre-administration of monoclonal antibody 2H5 was used.

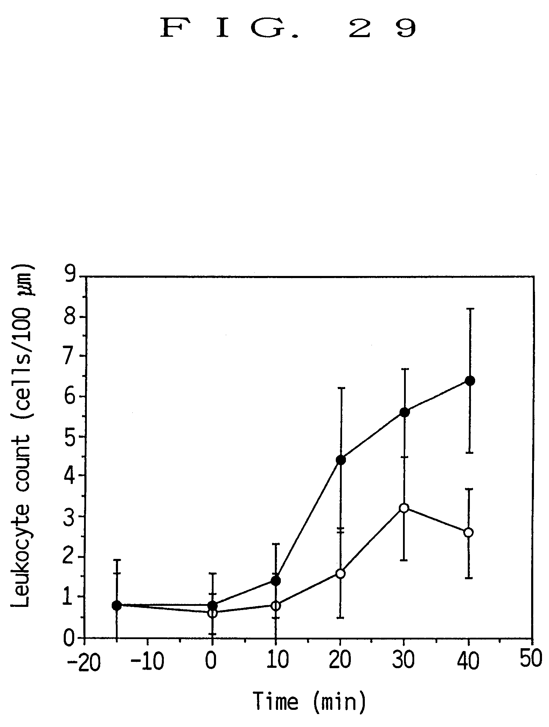

The obtained leukocyte counts were converted to the values per 100 µm of post capillary venule of mesentery, and standard deviation was determined, the results of which are shown in Fig. 29.

As a result, it was proved that the monoclonal antibody 2H5 of the present invention significantly inhibited the adhesion of leukocytes to vascular endothelial cell membrane at the inflamed area of nonlymphoid tissues in inflamed animal models, thereby suggesting the usefulness of said monoclonal antibody as an anti-inflammatory agent.

### Example 9

### Inhibitory effect of monoclonal antibody 2H5 on rolling of leukocytes in rat models with inflammation induced by histamine

Using rat models with inflammation induced by histamine, inhibitory effect of monoclonal antibody 2H5 on rolling of leukocytes on vascular endothelial cell membrane was examined.

WKYs rats (12 weeks old, male, 290-320 g, 4 rats) were anesthetized by an intramuscular injection of sodium pentobarbital (40 mg/kg) and stabilized for 30 minutes. The abdomen was incised by surgery and mesentery was pulled out. After perfusion under 95% nitrogen/5% oxygen concentrations, the mesentery was placed under orthostereobiomicroscopy. Then, monoclonal antibody 2H5 [monoclonal antibody 2H5 (600 µl/diluted with physiological saline (900 µl)] was administered at a concentration of 2 mg/kg from femoral vein over 15 minutes. At 15 minutes after the completion of administration of monoclonal antibody 2H5, histamine (10 µM) solution was added to the mesentery. The kinetics of leukocytes and erythrocytes in the blood stream of post capillary venule of the mesentery (30-40 µm) placed under the microscopy was videotaped from before the administration of monoclonal antibody 2H5. The flow rate of leukocytes flowing while rolling on vascular endothelial cell membrane during the period of from the completion of histamine administration to 30 minutes thereafter was determined as a relative speed (%) to the flow rate of erythrocytes. As a control, the group of 4 rats administered with histamine without pre-administration of monoclonal antibody 2H5 was used.

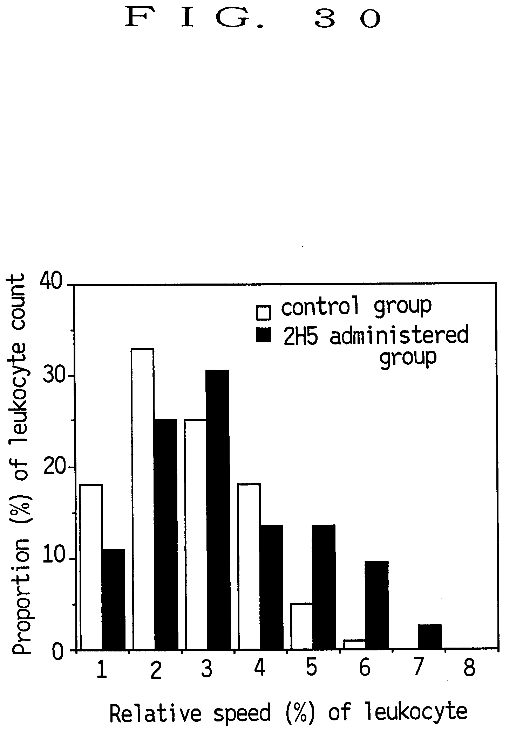

The results are shown in Fig. 30. Compared to the rolling speed in the control group, the group administered with monoclonal antibody 2H5 showed significant increase in the proportion of leukocytes having greater speed of rolling (rotational migration speed) on vascular endothelial cell membrane, relative to the flow rate of erythrocyte, whereby it was proved that the monoclonal antibody 2H5 of the present invention significantly inhibited the rolling (rotational migration) of leukocytes on vascular endothelial cells at the inflamed area of nonlymphoid tissues in inflamed animal models, thereby suggesting the usefulness of said monoclonal antibody as an anti-inflammatory agent.

### Example 10

### Inhibitory effect of monoclonal antibody 2H5 on tissue infiltration of leukocytes in rat models with peritonitis induced by thioglycolate

Using rat models with peritonitis induced by thioglycolate, inhibitory effect of monoclonal antibody 2H5 prepared in Example 2 on tissue infiltration of leukocytes was examined.

Monoclonal antibody 2H5 (2 mg/kg/ml physiological saline) was intravenously injected to Donryo rat (6 weeks old, 6 per group) via tail vein under ether anesthesia. Immediately thereafter, aqueous thioglycolate (29 g/ℓ) solution (5 ml) prepared by dissolving in distilled water was intraperitoneally administered. Five hours later, the rats were killed by blood loss upon carotid section and the abdomen was incised. The abdominal cavity was washed with 0.5% BSA/heparin (100 U/ml)/D-PBS solution (5 ml) and the washing was recovered. The infiltrated leukocytes in the recovered washing were counted with an automatic cytometer (Toa Iyo Denshi Corp.).

As a positive control, the group administered with thioglycolate alone without pre-administration of monoclonal antibody 2H5 was used, and as a negative control, the group which underwent the same procedure without administration of monoclonal antibody 2H5 or thioglycolate was used.

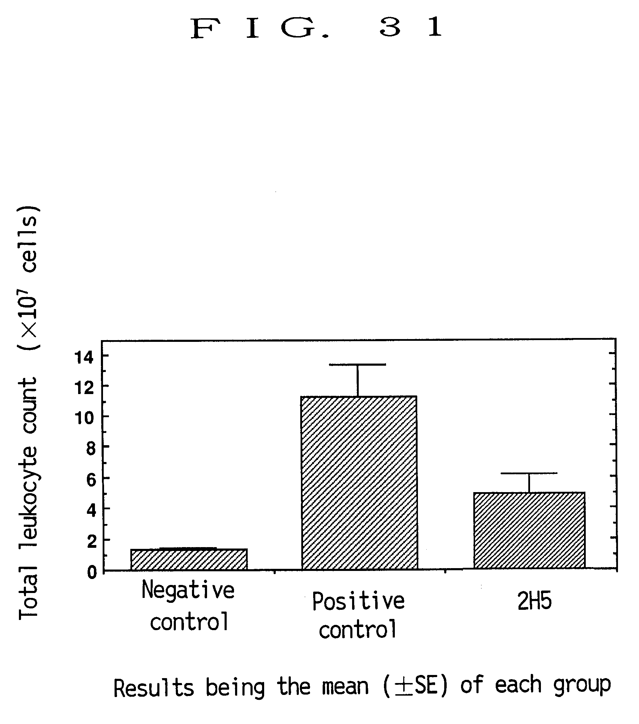

The results are shown in Fig. 31.

It was proved that the monoclonal antibody 2H5 of the present invention significantly inhibited infiltration of leukocytes into extravascular tissues at the inflamed area of nonlymphoid tissues in inflamed animal models (63% inhibition in this test), thereby suggesting the usefulness of said monoclonal antibody as an anti-inflammatory agent.

### Example 11

### Specificity of reactivity of monoclonal antibody 2H5 to sialyl Lewis X sugar chain antigen (sialylated sugar chain antigen)

In Examples 3-6, the reactivity of monoclonal antibody 2H5 to sialyl Lewis X sugar chain antigen was proved.

In this Example 11, the specificity of reactivity of monoclonal antibody 2H5 to sialyl Lewis X sugar chain antigen was examined by comparative examination of the reactivity to leukocytes (neutrophil) wherein terminal sialic acid of sialyl Lewis X sugar chain antigen (sialylated sugar chain antigen) had been removed by an enzyme treatment with neuraminidase, and the reactivity to untreated leukocytes (neutrophil).

Blood was obtained from healthy human volunteer, WKY rat and BALB/c mouse, and erythrocytes were hemolyzed with a non-isotonic solution to give leukocytes. The leukocytes (1×10⁶) treated with 0.1 U/ml neuraminidase (Nacalai Tesque) at 37°C for one hour and untreated leukocytes (1×10⁶) were each reacted with monoclonal antibody 2H5, then with FITC-labeled anti-mouse IgM (Cappel), and fluorescence intensity of stained neutrophil fraction was determined by the use of EPICS-Elite flow cytometer.

Using, as a control, mouse IgM (Chemcon) instead of monoclonal antibody 2H5, a similar determination was performed.

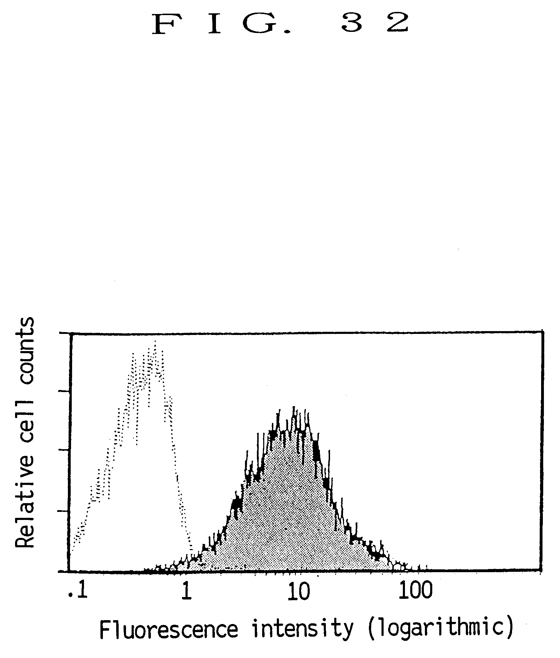

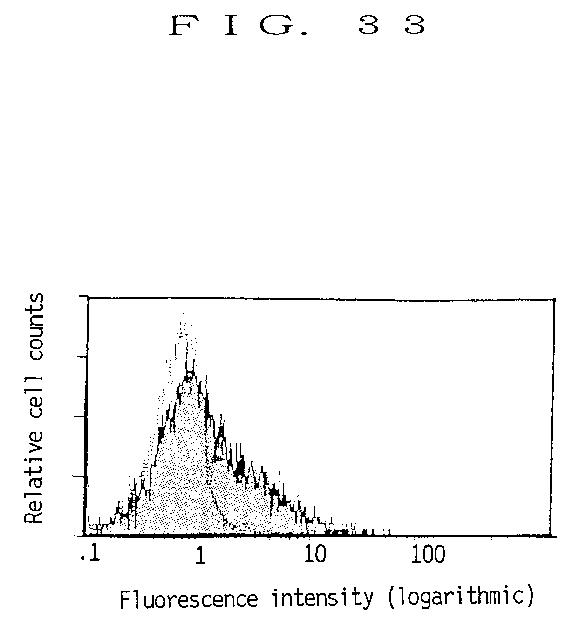

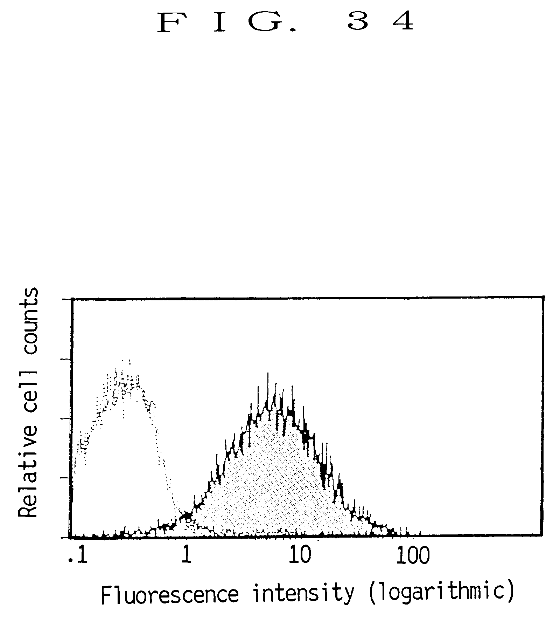

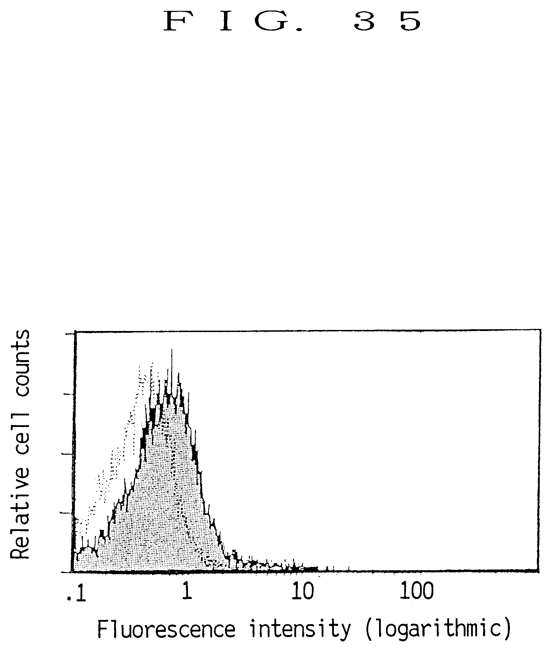

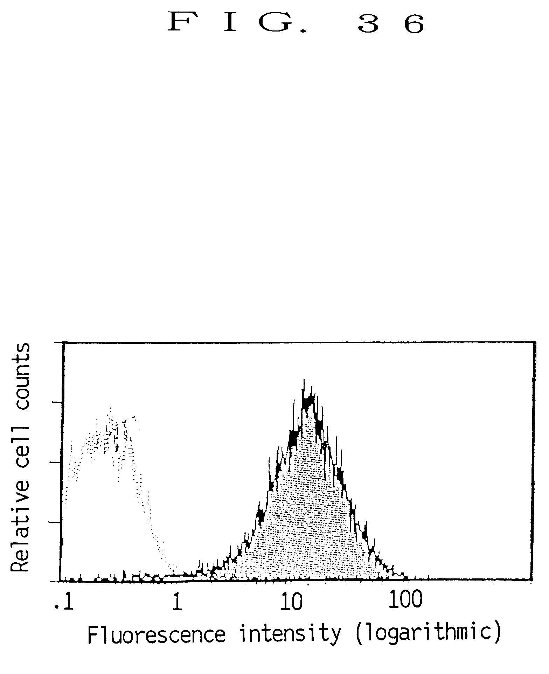

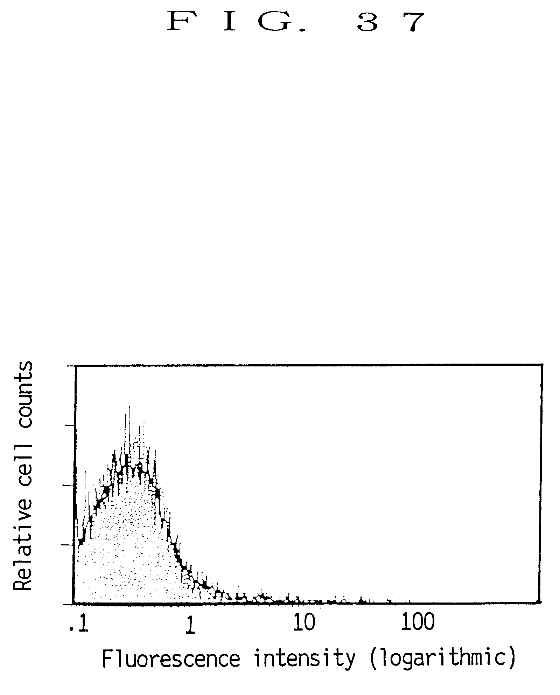

The results are shown in Figs. 32 to 37. Therefrom it was proved that the monoclonal antibody 2H5 of the present invention specifically showed reactivity to leukocytes having a sialylated sugar chain (sialyl Lewis X sugar chain), but did not show reactivity to leukocytes wherein terminal sialic acid had been removed by a neuraminidase treatment.

In Example 3, the absence of reactivity of monoclonal antibody 2H5 of the present invention to sialyl Lewis **a** sugar chain and the presence of reactivity to sialyl Lewis X sugar chain were already proved. The results of Example 3 and the results of the above-mentioned Example 11 proved that the monoclonal antibody 2H5 of the present invention had specific reactivity to sialyl Lewis X sugar chain alone.

### Example 12

### Anti-inflammatory effect of monoclonal antibody 2H5 in reperfusion injury after myocardial ischemia

Using rat models with reperfusion injury after myocardial ischemia, the anti-inflammatory effect of monoclonal antibody 2H5 on reperfusion injury after myocardial ischemia was examined.

### (1) Surgery

Wistar rats (7-10 weeks old, 200-280 g) were anesthetized by an intraperitoneal injection of sodium pentobarbital (25 mg/kg). An artificial respiration device (SN-480-7, Shinano Seisakusho) was intubed to send the in-room air (ventilation 20 ml/kg/60 min) for oxygen supply. The left side of the chest was incised, pericardium was excised and the chest was pressed with clamp to cause coronary occlusion. Thirty minutes later, the clamp was removed for reperfusion of blood. Standard second induction electrocardiogram was continuously recorded while checking the changes in ST segment level of electrocardiogram and the color changes of cardiac muscle, whereby ischemia and reperfusion of the entire cardiac muscle were confirmed. The chest was sutured and the rat came out from surgery anesthesia.

### (2) Antibody treatment

In the surgery of (1), the rats were divided into three groups of a group administered with monoclonal antibody 2H5, a group administered with rat IgM (Jeokson Immnoresearch Laboratories) and a group administered with PBS (phosphate-buffered saline). Monoclonal antibody 2H5, rat IgM and PBS were administered to each group including 8 rats per group at a concentration of 2 mg/kg body weight from femoral vein 5 minutes before coronary occlusion. At 48 hours after reperfusion, left ventricle of each heart was excised, washed with PBS solution and cut into 6 equal circular slices. Each tissue section was incubated for 4 minutes in 0.2 M Tris buffer (pH 8.0, 26°C) containing 1% triphenyltetrazol chloride (TTC). In this treatment, the region which became necrotic by inflammation did not develop color, while non-necrotic region developed red brick color.

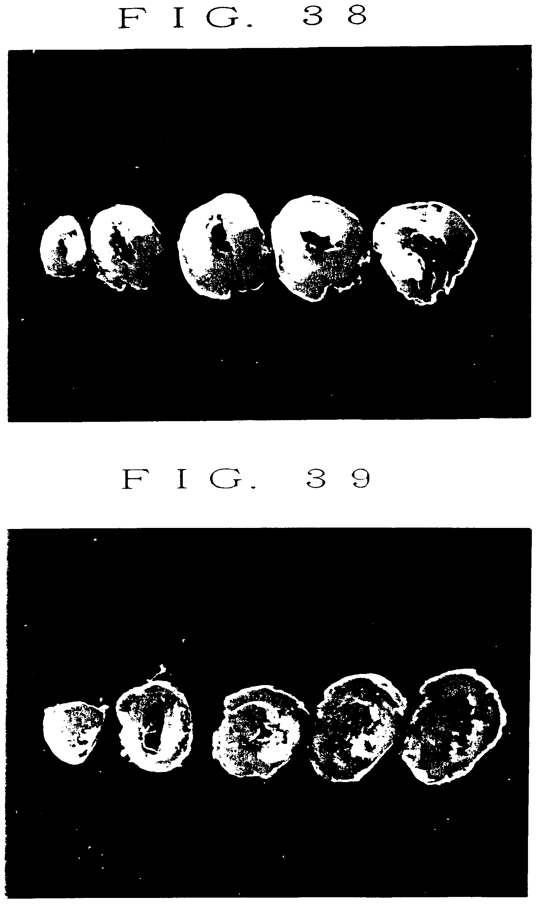

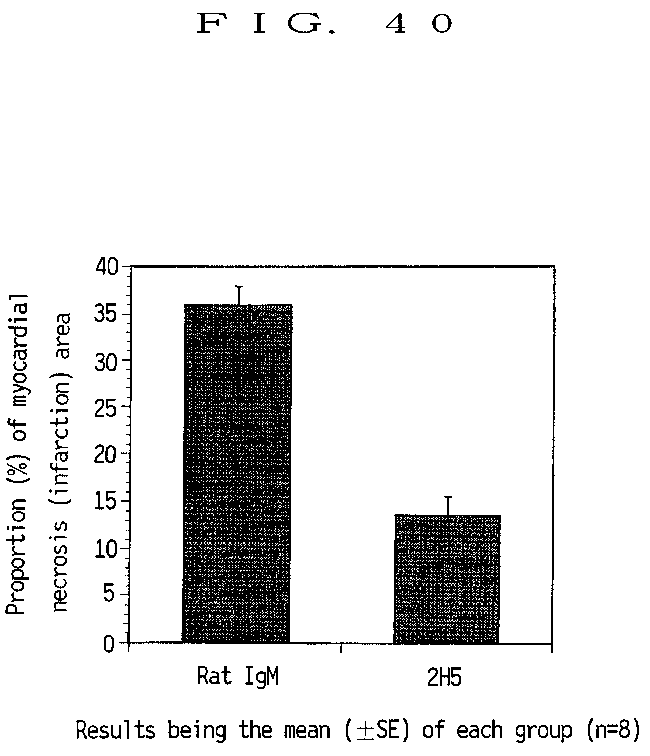

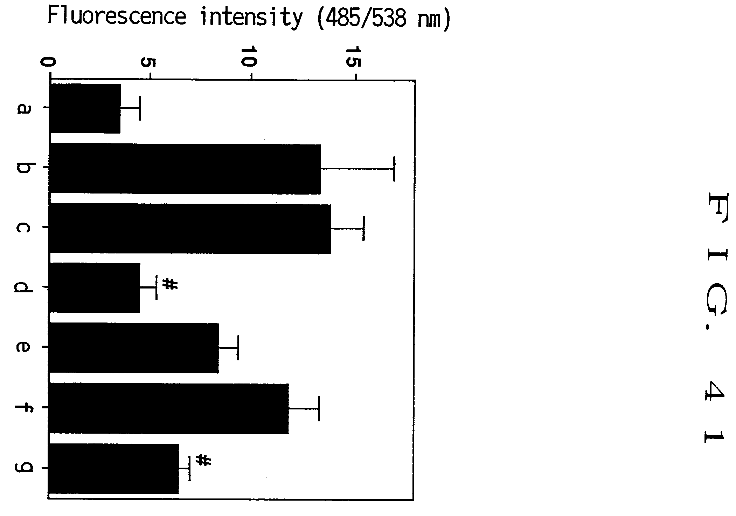

The results are shown in Figs. 38-40. From these results, it was proved that the monoclonal antibody 2H5 had significant anti-inflammatory effect on inflammation caused by reperfusion injury after myocardial ischemia.

### Example 13

### Inhibitory effects of monoclonal antibody 2H5 on cell adhesion via E-selectin and P-selectin

The following test was done to examine the inhibitory effects of monoclonal antibody 2H5 on cell adhesion of leukocytes to vascular endothelial cells via E-selectin and P-selectin on vascular endothelial cell membrane.

HL-60 cells (ATCC CCL-240), which is a human peripheral blood promyelocyte cell line, was labeled with 10 µM 2',7'-bis(carboxyethyl)carboxyfluoreintetraacetoxymethyl ester (Molecular Probes) at 37°C for 30 minutes, washed with RPMI1640 medium, and suspended in RPMI1640 containing 1% FCS. pME18s vector [supplied by Dr. Kazuo Maruyama, Tokyo University, see Experimental Medicine, Genetic Engineering Handbook, (separate volume), pp. 101-107 (1992)] into which cDNA (British Bio-Technology) encoding human E-selectin had been introduced, and CDM8 vector (Invitrogen) into which cDNA encoding human P-selectin had been inserted, were introduced into COS cells by electroporation. Twenty-four hours later, respective transformed cells were treated with trypsin, transferred to different 96 well culture plates and cultured for 48-72 hours. The plates were washed with RPMI1640 medium, and labeled HL-60 cells (0.1 ml of 2× 10⁶ cell/ml) and monoclonal antibody 2H5 (10 µg/ml) were added to each well of the plates, which was followed by incubation at 4°C for 20 minutes. Unbound cells were removed by gently washing with RPMI1640 medium 4 times. Bound cells were dissolved in 0.1% Nonidet P-40 solution (100 µl). Using fluoroscan II microplate fluorometer (Flow Laboratories), fluorescence intensity at 538 nm (excitation at 485 nm) was determined, based on which relative cell counts of each well were calculated. The statistical processing was done by Student's t-Test.

Using, as a control, mouse IgM (10 µg/ml, Chemcon) instead of monoclonal antibody 2H5, a similar determination was performed. For comparison, mouse anti-human P-selectin antibody (5 µg/ml, Biodesign) and mouse anti-human E-selectin antibody (10 µg/ml, British Biotechnology) were used in a similar determination.

The results are shown in Figs. 41 and 42. It was proved that the monoclonal antibody 2H5 significantly inhibited the adhesion of leukocytes to vascular endothelial cells via E-selectin and P-selectin on vascular endothelial cell membrane.

## Claims

1. A leukocyte rolling inhibitor comprising a monoclonal antibody having reactivity to a sialyl Lewis X sugar chain derived from a vascular endothelial cell membrane of a nonlymphoid tissue.

2. The inhibitor of claim 1, wherein the monoclonal antibody further has reactivity to a sialyl Lewis X sugar chain derived from a cell membrane of leukocyte.

3. The inhibitor of claim 1 or claim 2, wherein vascular endothelial cells of the nonlymphoid tissue are vascular endothelial cells of at least one tissue selected from the group consisting of brain, trachea, lung, liver, heart, pancreas, intestine, mesentery, kidney, skin and joint.

4. The inhibitor of claim 1 or claim 2, wherein vascular endothelial cells of the nonlymphoid tissue are vascular endothelial cells of at least one tissue selected from the group consisting of lung, liver, heart, pancreas, skin, joint and kidney.

5. The inhibitor of any one of claims 1 to 4, wherein the monoclonal antibody is produced by a hybridoma identified by the international deposit No. FERM BP-4525.

6. A leukocyte adhesion inhibitor comprising a monoclonal antibody having reactivity to a sialyl Lewis X sugar chain derived from a vascular endothelial cell membrane of a nonlymphoid tissue.

7. The inhibitor of claim 6, wherein the monoclonal antibody further has reactivity to a sialyl Lewis X sugar chain derived from a cell membrane of leukocyte.

8. The inhibitor of claim 6 or claim 7, wherein vascular endothelial cells of the nonlymphoid tissue are vascular endothelial cells of at least one tissue selected from the group consisting of brain, trachea, lung, liver, heart, pancreas, intestine, mesentery, kidney, skin and joint.

9. The inhibitor of claim 6 or claim 7, wherein vascular endothelial cells of the nonlymphoid tissue are vascular endothelial cells of at least one tissue selected from the group consisting of lung, liver, heart, pancreas, skin, joint and kidney.

10. The inhibitor of any one of claims 6 to 9, wherein the monoclonal antibody is produced by a hybridoma identified by the international deposit No. FERM BP-4525.

11. An inhibitor of infiltration of leukocytes into tissues, which comprises a monoclonal antibody having reactivity to a sialyl Lewis X sugar chain derived from a vascular endothelial cell membrane of a nonlymphoid tissue.

12. The inhibitor of claim 11, wherein the monoclonal antibody further has reactivity to a sialyl Lewis X sugar chain derived from a cell membrane of leukocyte.

13. The inhibitor of claim 11 or claim 12, wherein vascular endothelial cells of the nonlymphoid tissue are vascular endothelial cells of at least one tissue selected from the group consisting of brain, trachea, lung, liver, heart, pancreas, intestine, mesentery, kidney, skin and joint.

14. The inhibitor of claim 11 or claim 12, wherein vascular endothelial cells of the nonlymphoid tissue are vascular endothelial cells of at least one tissue selected from the group consisting of lung, liver, heart, pancreas, skin, joint and kidney.

15. The inhibitor of any one of claims 11 to 14, wherein the monoclonal antibody is produced by a hybridoma identified by the international deposit No. FERM BP-4525.

16. An anti-inflammatory agent comprising a monoclonal antibody having reactivity to a sialyl Lewis X sugar chain derived from a vascular endothelial cell membrane of a nonlymphoid tissue.

17. The agent of claim 16, wherein the monoclonal antibody further has reactivity to a sialyl Lewis X sugar chain derived from a cell membrane of leukocyte.

18. The agent of claim 16 or claim 17, wherein vascular endothelial cells of the nonlymphoid tissue are vascular endothelial cells of at least one tissue selected from the group consisting of brain, trachea, lung, liver, heart, pancreas, intestine, mesentery, kidney, skin and joint.

19. The agent of claim 16 or claim 17, wherein vascular endothelial cells of the nonlymphoid tissue are vascular endothelial cells of at least one tissue selected from the group consisting of lung, liver, heart, pancreas, skin, joint and kidney.

20. The agent of claim 16 or claim 17, wherein the inflammation is in brain, trachea, blood vessel, lung, liver, heart, pancreas, intestine, mesentery, kidney, skin or joint.

21. The agent of claim 16 or claim 17, wherein the inflammation is nephritis, pancreatitis, pneumonia, enteritis, dermatitis, hepatitis, arthritis or an inflammation caused by reperfusion injury after ischemia, immune rejection after transplantation, burns or multiple organ failure.

22. The agent of any one of claims 16 to 21, wherein the inflammation is an acute inflammation.

23. The anti-inflammatory agent of any one of claims 16 to 22, wherein the monoclonal antibody is produced by a hybridoma identified by the international deposit No. FERM BP-4525.
